# EUROPÄISCHE PATENTSCHRIFT

(11) **EP 3 824 016 B1**
(45) Veröffentlichungstag und Bekanntmachung des Hinweises auf die Patenterteilung: **02.04.2025**
(21) Anmeldenummer: 19749232.5
(22) Anmeldetag: 18.07.2019
(51) Int. Cl.: C08G 75/00, C08G 61/12

(54) **LICHTHÄRTENDE POLYMERELEKTRODE ZUR ABLEITUNG VON BIOSIGNALEN**
LIGHT-CURING POLYMER ELECTRODE FOR DERIVING BIOSIGNALS
ÉLECTRODE POLYMÈRE PHOTODURCISSABLE POUR LA DÉRIVATION DE BIOSIGNAUX

(30) Priorität: 21.07.2018 DE 202018003396 U
(43) Veröffentlichungstag der Anmeldung: 26.05.2021
(73) Patentinhaber: De Camp, Nora, 14129 Berlin (DE); Bergeler, Jürgen, 14482 Potsdam (DE)
(72) Erfinder: De Camp, Nora, 14129 Berlin (DE); Bergeler, Jürgen, 14482 Potsdam (DE)
(74) Vertreter: Hertin und Partner Rechts- und Patentanwälte PartG mbB
(86) Internationale Anmeldenummer: PCT/EP2019/069369
(87) Internationale Veröffentlichungsnummer: WO 2020/020741

(56) Entgegenhaltungen:
- EP-A1- 2 767 632
- WO-A1-2018/031821
- WO-A1-2018/111949
- DE-U1- 202016 003 395
- US-A1- 2018 014 780
- "Environmental Biosensors", 1 July 2011, INTECH, ISBN: 978-9-53-307486-3, article KATRINE KIILERICH-PEDERSEN: "Polymer Based Biosensors for Pathogen Diagnostics", pages: 193 - 212, XP055016998
- NORA VANESSA DE CAMP ET AL: "Light-cured polymer electrodes for non-invasive EEG recordings", SCIENTIFIC REPORTS, vol. 8, no. 1, 19 September 2018 (2018-09-19), XP055626197, DOI: 10.1038/s41598-018-32304-6

## Beschreibung

Die Erfindung betrifft eine lichthärtende Polymermischung zur Herstellung von Polymerelektroden, wobei die Polymermischung eine Mischung einer Poly-3,4-ethylendioxythiophen (PEDOT)-Verbindung mit einem auf Dimethacrylaten basierenden lichthärtenden Zahnzement umfasst. In weiteren Ausführungsformen betrifft die Erfindung zudem eine Polymerelektrode zur Ableitung von Biosignalen herstellbar mittels der lichthärtende Polymermischung sowie eine Vorrichtung zur Applikation der Polymerelektroden.

### HINTERGRUND UND STAND DER TECHNIK

Obwohl die nicht-invasive klinische Elektroenzephalographie (EEG) oder andere elektrophysiologischen Messmethoden etabliert sind, wird deren Verwendung in einem weiten Bereich möglicher Anwendungen durch technische Limitierungen verhindert. EEG-Verstärker haben zwar beispielsweise enorme Verbesserungen erfahren in Bezug auf deren Eingangswiderstand, Signal-Rausch-Verhältnis und Miniaturisierungsgrad. Die in den Verfahren eingesetzten Elektroden wurden jedoch nicht in gleichen Maßen verbessert.

Für EEG Ableitungen als einer elektrophysiologischen Messmethode bei der die höchste Leitfähigkeit der Elektroden benötigt wird, werden in der Regel haftende Elektrolytgele in Kombination mit Silber/Silberchlorid Elektroden eingesetzt. Silber/Silberchlorid Elektroden in Kombination mit einem Chlorid-haltigen leitenden Gel für EEG Ableitungen sind als nicht polarisierende EEG Elektrode der derzeitige Stand der Technik (Tallgren et al., 2005). Cup-Elektroden, die mit einem leitfähigen Gel befüllt werden, um einen niederohmigen Kontakt mit der Haut zu erreichen sind der derzeitige Goldstandard. Diese Elektroden sind relativ sperrig, und es ist nahezu unmöglich auf solchen Elektroden zu schlafen, ohne Druckstellen und Schmerzen zu verursachen. Ein Einsatz im neonatalen Bereich auf der Intensivstation, für die Schlafdiagnostik oder für Langzeitaufnahmen wird dadurch stark erschwert bzw. unmöglich gemacht (Lloyd et al. 2015, Löfhede et al. 2012).

Außerdem können Cup-Elektroden nur dann mit einer hinreichenden Stabilität platziert werden, wenn sie in einer Kopfkappe bzw. Elektrodenhaube integriert sind. Elektrodenhauben werden insbesondere für jüngere Kinder, Babys oder Neugeborene als belastend eingestuft und nicht empfohlen. Im Allgemeinen werden Elektrodenhauben von diesen auch nicht gut akzeptiert (Lloyd et al. 2015).

Ein weiterer Schwachpunkt dieser Systeme ist eine fehlende Langzeitstabilität der Messungen durch Austrocknung des Elektrolytgels und möglicher Kurzschlüsse durch verlaufendes Gel, besonders bei der Applikation einer hohen Anzahl von Elektroden. (Ferree et al., 2001, Chen et al., 2014).

Als alternative Lösungen sind Silber/Silberchloridelektroden auch als selbsthaftende Hydrogelelektroden oder Textilelektroden erhältlich, die für den Einmalgebrauch bestimmt sind (Löfhede et al. 2012, Alba et al. 2010). Diese Elektroden sind relativ großflächig und erlauben daher keine Ableitungen mit einer hohen Anzahl an Elektroden, wie sie beispielsweise für medizinische Montagen bei Frühgeborenen empfohlen wird. Die größere Oberfläche dieser Elektroden ist notwendig, um eine ausreichende Haftung auf der Haut und einen niedrigeren Kontaktwiderstand zu gewährleisten. Obwohl diese Elektroden flexibler sind, ist in den meisten Fällen eine Rasur erforderlich, da die Haare den notwendigen Hautkontakt beeinträchtigt Zudem können auch Hydrogelelektroden oder Textilelektroden austrocknen und somit ihre Funktionalität verlieren.

Als eine weitere Alternative wurden trockene Elektroden entwickelt (Krachunov & Casson, 2016). Aufgrund ihrer starren Materialeigenschaften ist es schwierig, einen stabilen Kontakt zur Haut zu erreichen, insbesondere falls sich der Proband oder Patient bewegt. Trockene Elektroden müssen daher mit Druck auf die Haut gepresst werden und können insbesondere bei Kindern Druckstellen verursachen. Auf Dauer sind trockene Elektroden unkomfortabel und eignen sich nicht für Langzeitmessungen bzw. werden insbesondere von jüngeren Kindern nicht akzeptiert.

Eine weitere technische Einschränkung ist ihr starkes kapazitives Verhalten, was dazu führt, dass keine Betrachtung des vollen Spektrums der EEG Wellen (*full band EEG*) möglich ist. Insbesondere kann keine Aufzeichnung einer sehr langsamen Hirnaktivität (Deltaband) erzielt werde, obwohl die langsamen Frequenzbänder eine wichtige Rolle bei der Hirnreifung spielen. Gerade das EEG von Frühgeborenen ist gekennzeichnet durch sehr langsame Delta-Schwingungen, die nur mit DC-gekoppelten Verstärkern aufgezeichnet werden können. Full-Band-EEGs (fbEEG) sind daher ein neuer Standard auf der neonatalen Intensivstationen (Vanhatalo et al., 2005).

Zur Aufzeichnung von Full-Band EEGs sind Elektroden mit nichtpolarisierenden Eigenschaften notwendig. Eine systematische Studie mit verschiedenen Metallelektroden zeigte, dass eine gesinterte Silber/ Silberchloridelektrode in Kombination mit einem Chlorid-haltigen Elektrodengel sich für ein Vollband-EEG am besten eignet. Daher werden trockene kapazitive Elektroden in der Regel nicht auf der neonatalen Intensivstation und bei Schlaganfall eingesetzt.

Aufgrund der bestehenden technischen Einschränkungen sind nicht-invasive EEG-Aufnahmen zudem von frei beweglichen, wachen Tieren nahezu unmöglich. Für wissenschaftliche Zwecke werden Elektroden daher implantiert (Lundt et al. 2016). Die meisten Tiere mit implantierten Elektroden müssen isoliert gehalten werden, um das Tier vor Verletzungen als Folge der Manipulation externer Komponenten zu schützen.

Es wurden einige Versuche unternommen, nicht-invasive EEG-Elektroden für wissenschaftliche Zwecke zu verwenden (Kim et al. 2017). Diese Systeme sind jedoch nicht auf frei bewegliche Tiere anwendbar. Stattdessen werden die Tiere während der tierärztlichen Betreuung sediert, was wiederum die Diagnose erschwert (James et al. 2011).

In einer aktuellen Studie wurden subdermale Elektroden für eine Epilepsiediagnostik bei unsedierten Hunden verwendet (James et al. 2017). Die Aufzeichnungsqualität leidet jedoch aufgrund häufiger Artefakte unterschiedlicher Herkunft.

Im Stand der Technik gibt es mithin einen Bedarf an verbesserten Elektroden im Hinblick auf die Handhabung, den Tragekomfort sowie die Aufzeichnungsqualität insbesondere für nicht-invasive EEGs.

Auch andere elektrophysiologische Messungen wie beispielsweise EKGs könnten von derart verbesserten Elektroden profitierten. Beispielsweise ist es im Rahmen von MRT Messungen notwendig, spezielle nicht-metallische Karbonelektroden zu verwenden, damit diese nicht mit den Magnetfeldern interferieren. Die Karbonelektroden für EKG Messungen im MRT haften jedoch schlecht auf leicht behaarter Haut. Auch sind die Kabel relativ starr. Beide Faktoren begünstigen Störungen im EKG. Da das EKG-Signal als Trigger für die MRT Bildgebung genutzt wird, verzögern EKG-Störungen die MRT-Messungen, wodurch signifikant erhöhte Kosten sowie Belastungen für den Patienten durch längere Messzeiten entstehen.

Auch für EKG-Elektroden, insbesondere für Anwendungen zur MRT-Bildgebung, gibt es daher einen Bedarf an Verbesserungen.

Die Entwicklung von Elektroden zur Ableitung von Biosignalen fokussierte sich traditionell auf Metallelektroden, wie beispielsweise Gold- oder Silberelektroden. In den letzten Jahren wurden verschiedene nichtmetallische leitende Materialien entwickelt (Balint et al. 2014).

Poly(3,4-ethylendioxythiophen) (PEDOT) ist ein leitfähiges Polymer, welcher primär zur Herstellung von Solarzellen verwandt wird (Li et al. 2017). Auch als Kandidat für medizinische Biosensoren wurde PEDOT bereits diskutiert (Rozlosnik et al. 2009, Kiilerich-Pedersen et al. 2011 ).Der mögliche Einsatz von PEDOT zur Herstellung von Polymerelektroden, welche sich beispielsweise für nicht-invasive EEGs eignen ist nicht weit erforscht.

EP 2 767 632 A1 betrifft die Herstellung von leitfähigen Kabeln bzw. Fasern insbesondere zur Kontaktierung von Elektroden. EP 2 767 632 A1 schlägt insbesondere zu dem Zweck vor Basisfasern mit PEDOT-PSS als leitfähigem Polymer zu imprägnieren. Für die Bereitstellung von Elektroden wird u.a. auch eine Beschichtung von PET-Gewebe mit PEDOT-PSS offenbart.

US 2018/014780 A1 betrifft die Herstellung von leitfähigen Polymerelektroden, insbesondere aus leitfähigem Polymergewebe beispielsweise für Sportanwendungen oder zur Gesundheitsüberwachung. In bevorzugten Ausführungsformen wird als leitfähiger Polymer PEDOT:PSS vorgeschlagen.

Aus der DE 20 2016 003 395 ist ein weiterer Ansatz bekannt. So schlägt DE 20 2016 003 395 eine lichthärtende Polymerelektrode auf Basis einer Mischung aus PEDOT und einem auf Methylmethacrylat basierenden Zahnzement vor. Die offenbarte Polymerelektrode lässt sich jedoch schlecht von der Haut entfernen und weist zudem höhere Impedanzen insbesondere auf Haut auf, welche zu verminderter Aufzeichnungsqualität führen. Auch kann keine Langzeitstabilität für niedrige Impedanzen erreicht werden. Zudem erschwert die Konsistenz der in der DE 20 2016 003 395 beschriebenen Polymerelektrode eine schmerzfreie Entfernung von der Haut, insbesondere bei einer Applikation auf einer Haut mit Haaren bzw. Flaum, wie dies bei Neugeborenen der Fall ist.

WO 2018/111949 A1 offenbart eine biokompatible Elektrode, welche in flüssiger Form in Gewebestrukturen injiziert werden kann, um dort auszuhärten. WO 2018/111949 A1 offenbart eine Vielzahl verschiedener Zusammensetzungen. Zur Bereitstellung eines härtbaren flüssigen Trägers werden Verbundharze aus dem Zahnbereich auf Dimethacrylat-Basis oder Knochenzement beschrieben.

WO 2018/031821 A1 betrifft härtbare Verbundharze mit strukturierten Mikropartikeln, u.a. für zahnmedizinische Anwendungen. In einer Ausführungsform umfasst das härtbare Harz einen Acrylat-basierten Polymer u.a. TEGDMA, BisGMA oder auch UDMA. Die strukturierten Mikropartikel können bevorzugt aus Oxiden, beispielsweise Siliciumdioxid, hergestellt werden. Bevorzugt erfolgt die Applikation der Verbundharze auf einem Zahn als Substrat.

### AUFGABE DER ERFINDUNG

Eine Aufgabe der Erfindung war es, eine Elektrode sowie Mittel bzw. Verfahren zur Herstellung derselben bereitzustellen, welche die Nachteile des Standes der Technik beseitigen. Insbesondere war es eine Aufgabe der Erfindung eine Elektrode bereitzustellen, welche sich durch eine erhöhten Tragekomfort, eine einfache Handhabbarkeit, zuverlässige Aufbringung und hohe Aufzeichnungsqualitiät auszeichnet. Bevorzugt sollten die bereitgestellten Elektroden somit auch schonende Langzeitableitungen, beispielsweise für Langzeit-EEGs als Routineuntersuchung für frühgeborene Kinder, ermöglichen. Auch eine flexible Einsatzfähigkeit zur Ableitung anderer Biosignale, wie beispielsweise für EKG, im Rahmen einer MRT Untersuchung wäre wünschenswert. Zudem sollte eine schmerzfreie Applikation unter Haaren bzw. Flaum, wie Frühgeborene sie aufweisen, ermöglicht werden.

### ZUSAMMENFASSUNG DER ERFINDUNG

Die Aufgabe der Erfindung wird durch die unabhängigen Ansprüche gelöst. Die abhängigen Ansprüche betreffend bevorzugte Ausführungsformen der Erfindung.

In einem Aspekt betrifft die Erfindung eine lichthärtende Polymermischung zur Herstellung von Polymerelektroden, wobei die Polymermischung eine Mischung von Poly-3,4-ethylendioxythiophen (PEDOT) mit einem auf Dimethacrylaten basierenden Zahnzement umfasst.

Eine derartige Polymermischung erlaubt die Herstellung einer niederohmigen und biokompatiblen Elektrode mit einer langfristig stabilen Haftung auf der Haut auf besonders einfache Weise. Hierzu kann in einem Arbeitsgang, das leitfähige, gelartige Polymer als dünner Film auf die Haut geklebt werden. Eine Bestrahlung des Polymers mit Licht, bevorzugte blauem Licht, führt zur Härtung der Polymermischung und damit zu einem haftenden und zugleich leitfähigen Hautkontakt. Für standardmäßige Zahnzemente eignen sich beispielsweise handelsübliche Blaulichtquellen aus der Zahnarztpraxis.

Poly-3,4-ethylendioxythiophen (PEDOT), in einigen Publikation auch PEDT oder PDT, bezeichnet vorzugsweise ein elektrisch leitfähiges Polymer auf Thiophenbasis. Es besteht aus 2,5-verknüpften 3,4-Ethylendioxythiophen-Einheiten (EDOT). Das Polymer besitzt vorzugsweise ein vollständig durchkonjugiertes π-System an Doppelbindungen. Die automatisch eintretende "Dotierung" während der Polymerisation zu PEDOT durch einen Dotant ermöglicht die Leitfähigkeit. Im oxidierten, leitfähigen Zustand werden die positiven Ladungen der Löcher in der konjugierten Kette durch Anionen kompensiert. PEDOT selbst ist vorteilhafterweise ungiftig (Miriani et al. 2008).

Unter dem Begriff PEDOT-Verbindung sollen sämtlich Verbindungen auf Basis eines PEDOT umfasst sein. Insbesondere, kann die PEDOT-Verbindung beispielsweise PEDOT, PEDOT:PSS; PEDOT:Tos, PEDOT-TMA oder PEDOT:CI sein. Besonders bevorzugt handelt es sich bei der PEDOT-Verbindung um PEDOT:PSS.

PEDOT:PSS bzw. Poly(3,4-ethylendioxythiophen) Polystyrolsulfonat bezeichnet bevorzugt eine Polymermischung aus zwei Ionomeren. Eine Komponente in dieser Mischung besteht aus Natriumpolystyrolsulfonat, welches ein sulfoniertes Polystyrol ist. Ein Teil der Sulfonylgruppen ist deprotoniert und trägt eine negative Ladung. Die andere Komponente ist bevorzugt Poly(3,4-ethylendioxythiophen) oder PEDOT, welches als konjugiertes Polymer positive Ladungen trägt. Zusammen bilden die geladenen Makromoleküle ein makromolekulares Salz.

PEDOT:PSS kann beispielsweise über die Oxidation von EDOT durch katalytische Mengen an Eisen(III)-sulfat in Wasser dargestellt werden. Die Reoxidation von Eisen wird durch Natriumpersulfat gegeben. Zusätzlich kann der Polyelektrolyt Natrium-Polystyrolsulfonat zugegeben werden, der neben der Dotierung des PEDOTs auch für dessen Wasserlöslichkeit sorgt. Nachdem eine tiefblaue Dispersion entstanden ist, wird das Reaktionsgemisch mit Hilfe von Anionen- und Kationentauscherharzen aufgereinigt. Die wässrige PEDOT:PSS Dispersion erreicht elektrische Leitfähigkeiten von ca. 0.1 - 2 S/cm. Durch bevorzugt hochsiedende Lösungsmittel wie DMSO oder Ethylenglycol kann die Leitfähigkeit auf bis zu 500 S/cm erhöht werden. Die Leitfähigkeitserhöhung ist auf die Separation von PEDOT und PSS bei einer Filmbildung zurückzuführen. Zudem handelt es sich bei einer wässrigen PEDOT:PSS Dispersion um eine einsatzfertige wässrige Suspension.

Aufgrund der guten Leitfähigkeit ist es bekannt PEDOT bzw. PEDOT-Verbindungen zur Herstellung von organischen Solarzellen zu verwenden. Teilweise wurde auch bereits eine Anwendung für medizinische Biosensoren diskutiert (Rozlosnik et al. 2009).

Eine Nutzung von PEDOT-Verbindungen zur Herstellung von Polymerelektroden, ist aus dem Stand der Technik weitestgehend unbekannt. In der DE 20 2016 003 395 wurde ein erster Ansatz offenbart, in welchem eine Polymermischung aus PEDOT zusammen mit einen auf Methylmethacrylat basierenden Zahnzement vorgeschlagen wird.

In weitergehenden Tests hat sich jedoch gezeigt, dass bei Verwendung von Methylmethacrylat basierenden Zahnzement, beispielsweise auf Basis von PMMA, zur Herstellung der Polymerelektroden, oft hohe Widerstandswerte von 40 kOhm oder mehr aufweisen und somit für viele Anwendungen nicht gut geeignet sind. Auch ist das Ablöseverhalten von Polymerelektroden unter Verwendung von Methylmethacrylat basierenden Zahnzements beeinträchtigt und führt zu unerwünschten Hautirritation. Außerdem lässt sich die Elektrode aufgrund ihrer Konsistenz nicht unter Haaren applizieren und danach schmerzfrei entfernen.

Überraschenderweise führt die Mischung einer PEDOT-Verbindung mit einem lichthärtendem Zahnzement auf Basis von Dimethacrylaten zu einer Polymermischung, welche diese Nachteile beseitigt und die Bereitstellung einer niederohmigen sowie flexibel und einfach zu applizierenden Polymerelektrode erlaubt. Eine Applikation unter Haaren bzw. Fell sowie die schmerzfreie Entfernung sind mit dieser Mischung möglich. Hierdurch ist die Polymerelektrode insbesondere für die Anwendung für Frühgeborenen geeignet, welche eine behaarte Kopfhaut aufweisen. Auch für Tiere mit einem Fell lassen sich vorteilhafterweise Biosignale ableiten, ohne dass eine aufwendige Rasur erforderlich wäre, noch Schmerzen auftreten.

Im Sinne der Erfindung meint ein "lichthärtendes Zahnzement" insbesondere ein lichthärtendes Glasionomerzement, lichthärtendes (Zahn-)Komposit oder Kombination selbiger, welche als Füllungsmaterialien in der Zahnheilkunde zum Einsatz kommt. In bevorzugten Ausführungsformen handelt es sich um lichthärtende kunststoffmodifizierte Glasionomerzemente (KGIZ), lichthärtende Komposite oder lichthärtende Glasionomer-Komposit-Zemente.

In einer bevorzugten Ausführungsform betrifft die Erfindung somit auch eine Polymermischung zur Herstellung von Polymerelektroden, wobei die Polymermischung eine Mischung einer Poly-3,4-ethylendioxythiophen (PEDOT)-Verbindung mit einem lichthärtenden Glasionomerzement, einem lichthärtenden Komposit und/oder einem lichthärtenden Kompomer, wobei das Glasionomerzement, der Komposit und/oder der Kompomer Dimethacrylate umfasst.

Auf dem Fachgebiet der Zahnmedizin sind dies gängige Begriffe für Werkstoffe bzw. Werkstoffklassen, welcher ein Fachmann kennt oder in der einschlägigen Fachliteratur nachsehen kann (vgl. u.a. Frankenberger et al. 1999).

Konventionelle Glasionomerzemente (GIZ) bestehen bevorzugt aus einem Gemisch aus Carbonsäuren (z. B. Polyacrylsäure oder deren Kopolymere), anorganischen Füllkörpern (Kalzium-Aluminium-Silikat-Glas) und destilliertem Wasser, das durch eine Säure-Basen-Reaktion aushärtet (vgl. u.a. Guggenberger et al. 1998). Kommerziell vertriebene Beispiele sind u.a. Ketac Fil^{®} der Firma ESPE, Fuji II^{®} der Firma GC oder Aqua Ionofil^{®} der Firma Voco.

Um eine verbesserte Handhabung zu gewährleisten wurden Methacrylatgruppen an die Polyacrylsäuren konventioneller Glasionomerzemente angefügt. Hierdurch kann eine initiale Photopolymerisation und damit längere Verarbeitung erreicht werden. Die Klasse derart modifizierter Glasionomerzemente wird auch als kunststoffmodifizierten Glasionomerzemente (KGIZ) bezeichnet. Hauptbestandtteile der kunststoffmodifizierten GIZ (KGIZ) sind die methacrylierte Polyacrylsäure, ein photopolymerisierbares Monomer beispielsweise *HEMA* (Hydroxy-Ethyl-Methacrylat), ionisierbare Gläser und Wasser. Nach dem Anmischen beider Komponenten kann mittels einer Lichtaktivierung eine Polymerisation der Methacrylatgruppe mit HEMA, eine Eigenpolymerisation von HEMA oder eine Eigenpolymerisation der reaktionsfähigen Methacrylat-Seitenkette auslösen.

Nach der Lichthärtung kann bei diesen Zweikomponentenmaterialien wie bei den konventionellen Glasionomerzemente eine Säure-Base-Reaktion ablaufen (Attin et al. 1996). Kommerziell vertrieben Beispiele der Werkstoffgruppe sind u.a. Photac^{®} Fil Quick der Firma ESPE, Vitremer^{®} der Firma 3M Dental Products, St. Paul, USA oder und Fuji^{®} II LC der Firma GC, B-Leuven).

(Zahn)Komposite sind bevorzugt zahnfarbene plastische Füllungsmaterialien für die zahnärztliche Behandlung. Komposite bestehen bevorzugt aus als grundsätzliche Komponenten aus einer organischen Matrix bzw. organische Phase, anorganischen Füllstoffen und optional Verbundstoffen (vgl. Zimmerli et al. 2010).

Die organische Matrix basiert bevorzugt auf Kunstoffen auf Acrylatbasis, bevorzugt Methacrylaten, welche zur Strahlenhärtung verwandt werden. Hierbei kommen bevorzugt Methylmethacrylat (MMA) bzw. Polymethylmethacrylat (PMMA) als auch Dimethacrylate (z.B. Bisphenolglycidylmethacrylat (BisGMA) oder Urethan-Dimethacrylat (UDMA)) als Basismonomere zum Einsatz.

Darüber hinaus kann die organische Phase noch weitere Bestandteile enthalten. Beispielsweise Verdünner zur besseren Verarbeitbarkeit (z.B. Comonomere TEGDMA und EGDMA oder andere Mono-, Di- und Triacrylate), Initiatoren für eine Polymerisation bevorzugt nach Beleuchtung mit einem blauen Licht (z. B. Benzoylperoxid, Phenylpropandiol, Campherchinon), wobei z.B. durch die Freisetzung von freien Radikalen eine chemische Abbindereaktion bzw. Härte in Gang gesetzt wird, Akzeleratoren (z.B. Toluidin) zur Beschleunigung der Abbindereaktion, (Photo-)Inhibitor (u.a. Hydrochinon), um zu verhindern, dass bereits durch normales Tageslicht eine Polymerisation ausgelöst oder andere Farb- und anderen Stabilisatoren (z.B. Benzophenon als UV-Stabilisator).

Anorganische Füllstoffe werden in (Zahn-)kompositen eingesetzt um Materialeigenschaften wie z.B. die Abrasionsbeständigkeit, Schrumpfung, Bruchfestigkeit etc. zu verbessern. Anorganische Füllstoffe können beispielsweise sein: Glas-, Keramik- oder Quarzteilchen. Nach der Größe ihrer Füllstoffe können Komposite unterteilt werden in Makrofüller, Mikrofüller oder Hybrid-Komposite.

Mikrofüllerkomposite enthalten beispielsweise splitter- oder kugelförmige Vorpolymerisate der organischen Matrix oder Siliciumdioxidteilchen. Hybridkomposite enthalten in der Regel 0,5 bis 10 µm große Glaspartikel und Zusätze, die das Material röntgensichtbar machen. Nano-Hybridkomposite umfassen Füllpartikeln im Nanobereich, teils mit konventionellen Füllern, teils mit Präpolymerisaten.

Die Eigenschaften der Komposite werden durch einen stabilen Verbund der organischen Matrix zu den Füllstoffen beeinflusst. Die Verbundphasenmolekule haben beispielsweise eine Silangruppe an einem und eine Methacrylatgruppe am anderen Ende und können so eine chemische Verbindung sowohl mit dem Füller als auch mit der Harzmatrix eingehen. Durch eine Silanisierung kann eine erhöhte Festigkeit erzielt werden.

Kommerziell vertriebene Beispiele von Kompositen sind beispielsweise Tetric EvoCeram^{®} als eine Hybridkomposit oder Filtek supreme XT^{®} als ein Nanokomposit.

Glasionomer-Komposit-Zement oder ein auch ein Compomer (bzw. Kompomer) sind bevorzugt Kombinationswerkstoffe aus Komposit und Glasionomerzement. Es handelt sich bei den Material bevorzugt um ein polyacryl-/polycarbonsäuremodifiziertes Komposit. Im Allgemeinen werden diese als Einkomponenten-Systeme geliefert werden. Sie umfassen insbesondere lichthärtende Monomeren, die Glas- und Quarzpartikel enthalten. Dadurch werden die guten Haftfähigkeiten des Glasionomer-Zements mit den Vorteilen des Komposits verbunden.

Kompomere bzw. Glasionomer-Komposit-Zemente enthalten insbesondere Monomere, Photoinitiatoren, Stabilisatoren, Farbpigmente und Füllkörper, die auch in Kompositen angewendet werden. Zusätzlich enthalten die Kompomere jedoch bevorzugt Dimethacrylatmonomere. Auch sind Füllkörper aus Barium-Aluminium-Fluorosilikat-Gläsern bevorzugt (Manhart et al. 2006).

Kompomere haben eine höhere Abriebfestigkeit als reine Glasionomerzemente, sind aber weniger abriebfest als Komposite. Bei Kompomeren ist wie bei Kompositen ein Adhäsiv für den sicheren Verbund mit der Zahnhartsubstanz notwendig und wird zumeist als Einkomponentensystem in der Zahnmedizin eingesetzt.

Neben verschiedenen polymerisierbaren Monomeren (z. B. UDMA) enthalten bevorzugte Kompomere Dikarbonsäuren, die aber im Unterschied zu den in herkömmlichen Glasionomerzementen verwendeten Karbonsäuren polymerisierfähige Doppelbindungen aufweisen. Aus der Glasionomertechnologie können bevorzugt reaktiven Fluoroaluminiumsilikatgläser im Kompomer eingesetzt werden (vgl. Zimmerli et al. 2010).

Kommerziell vertriebene Kompomere bzw. Glasionomer-Komposit-Zemente sind beispielsweise Dyract eXtra^{®} oder Ionoseal^{®} der Firma Voco.

Ein auf "Dimethacrylaten basierendes lichthärtendes Zahnzement" bezeichnet bevorzugt ein lichthärtendes Zahnzement, welches Dimethacrylate umfasst, bevorzugt ein lichthärtendes Zahnzement, welches zu 2 bis 60 Gew.-%, bevorzugt 5 bis 40 Gew.-%, besonders bevorzugt 20 bis 50 Gew.-% Dimethacrylate umfasst.

Methacrylsäure ist eine ungesättigte Carbonsäure, eine sogenannte Alkensäure, mit der Summenformel C₄H₆O₂ und wird als Ausgangsstoff zur Herstellung von Kunststoffen verwendet. Die Salze und Ester der Methacrylsäure werden als Methacrylate bezeichnet.

Dimethacrylate sind bevorzugt Methacrylate mit zwei polymerisierbaren Methacrylatgruppen. Dimethacrylate bezeichnen im Sinne der Erfindung bevorzugt Verbindungen mit genau zwei Methacrylatgruppen bzw. Methacrylsäureestergruppen pro Verbindung. Beispielhafte Dimethacrylate sind Bisphenol A-Glycidyldimethacrylat (BisGMA), Bisphenol A-dimethacrylat (BisMA), Urethandimethacrylate (UDMA), Tetraethylenglycoldimethacrylat (TETGDMA), Triethylenglykoldimethacrylat (TEGDMA), Ethylenglykoldimethacrylat (EGDMA) oder Diethylenglykoldimethacrylat (DEGDMA).

Dimethacrylate sind mithin durch das Vorhandensein zweier (polymerisierbarer) Methacrylatgruppen gekennzeichnet, wobei zwischen den Methacrylatgruppen unterschiedliche Verbindungsketten vorliegen können.

Wie die experimentellen Daten zeigen, können mit einem auf Dimethacrylat basierenden lichthärtenden Zahnzement in Verbindung mit einem PEDOT ausgezeichnete Ergebnisse erzielt werden. Zum einen sind sowohl auf einem Agarsubstrat als auf der Haut besonders geringe Widerstandswerte von 4 kOhm oder weniger messbar. Insbesondere nach einer Aushärtung der Polymermischung mittels Licht, bevorzugt blauem Licht, liegt über ein breites Frequenzspektrum eine ausgezeichnete Leitfähigkeit und geringen Widerstand vor. Dies erlaubt vorteilhafterweise einen Einsatz der Elektroden insbesondere für Full-Band-EEGs (fbEEG) beispielsweise zur Überwachung von Frühgeborenen.

In einer bevorzugten Ausführungsform der Erfindung ist die lichthärtende Polymermischung dadurch gekennzeichnet, dass die PEDOT-Verbindung mit dem auf Dimethacrylaten basierenden Zahnzement in einem Mischungsverhältnis zwischen 0.5:1 und 2:1 vor liegt. Auch Zwischenwerte aus den vorgenannten Bereichen können bevorzugt sein, beispielsweise ungefähr 0.6:1, 0.7:1, 0.8:1, 0.9:1, 1:1, 1.1:1, 1.2:1, 1.3:1, 1.4:1, 1.5:1, 1.6:1, 1.7:1, 1.8:1 oder 1.9:1. Ganz besonders bevorzugt ist das Mischungsverhältnis der PEDOT-Verbindung und eines auf Dimethacrylaten basierenden Zahnzement im Wesentlichen 1:1.

Begriffe wie im Wesentlichen, ungefähr, etwa, ca. etc. beschreiben bevorzugt einen Toleranzbereich von weniger als ± 20%, bevorzugt weniger als ± 10 %, noch stärker bevorzugt weniger als ± 5% und insbesondere weniger als ± 1%. Angaben von im Wesentlichen, ungefähr, etwa, ca. etc. offenbaren und umfassen stets auch den exakten genannten Wert.

Das Mischungsverhältnis erlaubt eine Anpassung der Leitfähigkeit sowie Impedanz und kann in Bezug auf die zu messenden Biosignalparameter (z.B. EEG, EKG, EMG) optimiert werden.

Die vorgenannten Parameter stellen besonders bevorzugte Bereiche dar und zeigen sowohl EEG, EKG als auch EMG ausgezeichnete Eigenschaften. Das besonders bevorzugte Mischungsverhältnis von im Wesentlichen 1:1 ist insbesondere für nicht-invasive EEG Messungen geeignet.

In einer bevorzugten Ausführungsform ist die lichthärtende Polymermischung dadurch gekennzeichnet, dass die PEDOT-Verbindung ein Poly-3,4-ethylendioxythiophen Polystyrolsulfonat (PEDOT:PSS) ist.

In bevorzugten Ausführungsformen kann die PEDOT-Verbindung zur Mischung mit dem Zahnzement als eine Mischung, Dispersion bzw. Tinte oder Paste bereitgestellt werden, welche neben der PEDOT-Verbindung weitere Additive umfasst. Beispielsweise kann die PEDOT-Verbindung als eine Dispersion umfassend die PEDOT-Verbindung sowie Tenside, Polymerbinder und/oder Lösungsmittel bereitgestellt werden.

In einer bevorzugten Ausführungsform umfasst die lichthärtende Polymermischung daher zusätzlich einen Polymerbinder, ein Tensid und/oder ein Lösungsmittel.

Polymerbinder bezeichnen bevorzugt Bindemittel, welche PEDOT-Polymer binden, indem sie diese aufnehmen, anlagern, zusammenhalten, vernetzen oder verkleben. Beispielhafte Polymerbinder sind u.a. Hydroxyethylcellulose (HEC), Polyacrylsäure (PAA) oder Polyvinylbutyral (PVB).

Tenside bezeichnen bevorzugt Substanzen, die die Oberflächenspannung einer Flüssigkeit oder die Grenzflächenspannung zwischen zwei Phasen herabsetzen und die Bildung von Dispersionen ermöglichen oder unterstützen bzw. als Lösungsvermittler wirken. Tenside gehören bevorzugt zur Molekülklasse der amphiphilen Substanzen. Molekular sind Tenside durch eine hydrophoben (unpolaren) Kohlenwasserstoffrest und einen hydrophilen (polaren) Molekülteil gekennzeichnet. Auf Basis des hydrophilen Molekülteiles bzw. der hydrophilen Gruppe erfolgt eine systematische Einteilung der Tenside. Anionische Tenside weisen als hydrophile Gruppen bevorzugt-COO⁻ (Carboxylate), -SO₃- (Sulfonate) oder -O-SO³⁻ (Sulfate) auf. Kationische Tenside besitzen bevorzugt -NR₄⁺ (Ammonium) als eine hydrophile Gruppe. Nichtionische Tenside sind bevorzugt durch -O-R (Polyether) oder -O-H (Polyalkohole) als hydrophilen Gruppen gekennzeichnet. Amphotere oder zwitterionische Tenside umfassen bevorzugt die hydrophilen Gruppen -NR₂⁺- (Ammonium) oder Carboxylat (-COO⁻).

In besonders bevorzugten Ausführungsformen sind die Tenside Silikon-Tenside (Polyethermodifizierte Polydimethylsiloxane) oder auch N-Alkylpyrrolidone (insbesondere 1-Octyl-2-pyrrolidon oder 1-Dodecyl-2-pyrrolidon).

Lösungsmittel bezeichnen bevorzugt Verbindungen, welche die PEDOT-Verbindung lösen oder verdünnen kann, ohne dass es dabei zu chemischen Reaktionen zwischen gelöstem Stoff und lösendem Stoff kommt, sodass die positiven Eigenschaften der PEDOT-Verbindung erhalten bleiben. Im Stand der Technik sind eine Vielzahl von Lösungsmittel bekannt, welche beispielsweise als aprotische oder protische Lösungsmittel klassifiziert werden können.

Eine nicht abschließende Liste von Lösungsmitteln umfasst beispielsweise Aceton, Acetonitril, Anilin, Anisol, Benzol, Benzonitril, Brombenzol, 1-Butanol, tert-Butylmethylether (MTBE), γ-Butyrolacton, Chinolin, Chlorbenzol, Chloroform, Cyclohexan, Dibutylether, Diethylenglycol, Diethylether, Dimethylacetamid, Dimethylformamid, Dimethylsulfoxid, 1,4-Dioxan, Eisessig, Essigsäureanhydrid, Essigsäureethylester, Ethanol, 1,2-Dichlorethan (Ethylendichlorid), Ethylenglycol, Ethylenglycoldimethylether, Formamid, n-Hexan, n-Heptan, 2-Propanol (Isopropylalkohol), Methanol, 3-Methyl-1-butanol (Isoamylalkohol), 2-Methyl-2-propanol (tert-Butanol), Methylenchlorid (Dichlormethan, DCM), Methylethylketon (Butanon), N-Methyl-2-pyrrolidon (NMP), N-Methylformamid, Nitrobenzol, Nitromethan, n-Pentan, Petrolether/Leichtbenzin, Piperidin, 1,2 Propandiol, Propanol, Propylencarbonat (4-Methyl-1,3-dioxol-2-on), Pyridin, Schwefelkohlenstoff, Sulfolan, Tetrachlorethen, Tetrachlorkohlenstoff, Tetrahydrofuran, Toluol, 1,1,1-Trichlorethan, Trichlorethen, Triethylamin, Triethylenglycol, Triethylenglycoldimethylether (Triglyme).

In besonders bevorzugten Ausführungsformen ist das Lösungsmittel ein 1,2 Propandiol, auch bekannt als Propylenglykol.

Die PEDOT-Verbindung, bevorzugt PEDOT:PSS, ist vorteilhafterweise in Form einer Dispersion bzw. Paste auch kommerziell erhältlich. Beispielsweise ist die Produktreihe Clevios^{™} PEDOT:PSS der Firma Heraus sehr gut geeignet zur Herstellung der lichthärtenden Polymermischung umfassend eine PEDOT:PSS-Verbindung. Ausgezeichnete Ergebnisse können mit Clevios^{™} PEDOT:PSS S V3 erzielt werden.

Die vorgenannten Clevios^{™} Produktreihe sind Sachangaben für besonders bevorzugte Dispersionen umfassend PEDOT:PSS, welche sich zu einer Beschaffenheitsangabe entwickelt haben und vom Fachmann als solche verstanden werden. Die Clevios^{™®} Produkte beziehen sich auf die chemischen Verbindungen, wie sie von der Firma Heraeus im Juni 2018 vertrieben und auf Datenblättern spezifiziert und veröffentlicht wurden.

In einer bevorzugten Ausführungsform ist das auf Dimethacrylaten basierende Zahnzement dadurch gekennzeichnet, dass dieses Bisphenol-A-(di)-methacrylate (BIS-GMA), Urethandimethacrylat, Hexandioldimethacrylate (HDDMA) und/oder Triethylenglycoldimethacrylat umfasst. Die vorgenannte Gruppe von Dimethacrylaten hat sich als besonders bevorzugt erwiesen. Unter Verwendung der bevorzugten Dimethacrylaten oder Kombinationen dieser können besonders zuverlässige niederohmige Polymerelektroden hergestellt werden, welche sich zudem durch eine gute Handhabung und einfaches Ablöseverhalten gekennzeichnet sind.

In besonders bevorzugten Ausführungsformen umfasst das auf Dimethacrylaten basierende Zahnzement 10-25 Gew.-% BIS-GMA, 5-10 Gew.-% Urethandimethacrylat, 5-10 Gew.-% HDDMA und 2,5 - 5 Gew.-% Triethylenglycoldimethacrylat.

In einer ganz besonders bevorzugten Ausführungsform ist das auf Dimethacrylaten basierende Zahnzement lonoseal.

Das Produkt Ionoseal^{®} der Firma Voco ist eine Sachangaben für ein besonders bevorzugtes Dimethacrylaten basierende Zahnzement, welche sich zu einer Beschaffenheitsangabe entwickelt hat und vom Fachmann als solche verstanden werden. Ionoseal^{®} bezieht sich auf die chemischen Verbindungen, wie sie von der Firma Voco im Juni 2018 vertrieben und auf Datenblättern spezifiziert und veröffentlicht wurden.

In einer weiteren bevorzugten Ausführungsform ist das auf Dimethacrylaten basierende Zahnzement Tetric EvoFlow^{®} der Firma Ivoclar Vivadent GmbH.

In einer bevorzugten Ausführungsform umfasst die lichthärtende Polymermischung als lichthärtende Komponente bzw. Initiatoren für eine Photopolymerisation Campherchinon, Acylphosphinoxid oder Ivocerin.

In einer Ausführungsform betrifft die Erfindung eine lichthärtende Polymermischung zur Herstellung einer Polymerelektrode umfassend
a) eine erste Mischung umfassend einer Poly-3,4-ethylendioxythiophen (PEDOT)-Verbindung, und
b) eine zweite Mischung umfassend Dimethacrylate als Monomere zur Bildung einer organischen Matrix

Die lichthärtende Polymermischung ist hierbei herstellbar durch einfaches Mischen der beiden Mischungen bei Raumtemperatur.

In bevorzugten Ausführungsformen liegt in der ersten Mischung die PEDOT-Verbindung in Form einer Dispersion vor, umfassend neben der PEDOT-Verbindung, zusätzlich einen Polymerbinder, Tenside und/oder Lösungsmittel vor.

In bevorzugten Ausführungsformen weist die zweite Mischung neben Dimethacrylaten als Monomere zur Bildung einer organischen Matrix anorganischen Füllmaterialien, bevorzugt Glas-, Keramik- oder Quarzteilchen, auf. Besonders bevorzugt kann die zweite Mischung weitere Zusatzstoffe umfassen, wie insbesondere lichthärtende Komponente bzw. Initiatoren für eine Photopolymerisation.

Der Fachmann erkennt, dass für die genannten Bestandteile der ersten und zweiten Mischung bevorzugten Ausführungsformen gelten.

Bei der ersten Mischung ist PEDOT:PSS eine besonders bevorzugte PEDOT-Verbindung. Vorteilhafterweise kann als eine besonders bevorzugte Ausführungsform für die erste Mischung die kommerziell erhältlichen Clevios^{™} PEDOT:PSS Produkte der Firma Hereaus verwandt werden.

Bei der zweiten Mischung handelt es sich bevorzugt um ein lichthärtendes Zahnzement auf Basis von Dimethacrylaten, wie ausführlich vorgehend beschrieben. Vorteilhafterweise kann als eine besonders bevorzugte Ausführungsform für die zweite Mischung das kommerziell erhältliche Ionoseal^{™} der Firma Voco verwandt werden.

In einem weiteren Aspekt betrifft die Erfindung eine Polymerelektrode zur Ableitung von Biosignalen von einer Haut umfassend eine lichthärtende Polymermischung gemäß der Erfindung oder bevorzugten Ausführungsformen davon.

In einem weiteren Aspekt betrifft die Erfindung eine Polymerelektrode zur Ableitung von Biosignalen von einer Haut herstellbar durch ein Verfahren umfassend
a. Bereitstellung einer lichthärtenden Polymermischung gemäß der Erfindung oder bevorzugten Ausführungsformen davon.
b. Auftragen der lichthärtenden Polymermischung auf ein Substrat, bevorzugt auf eine Haut, wobei es sich bevorzugt auch um eine behaarte Haut handeln kann,
c. Härtung der lichthärtenden Polymermischung mittels einer Lichtbestrahlung.

Im Sinne der Erfindung meinen Biosignale bevorzugt elektrische Biosignale, d.h. bevorzugt elektrische Spannungen bzw. Ströme als Ergebnis biologischer Aktivitäten. Biosignale basieren insbesondere auf den Änderungen eines elektrischen Stroms, die durch Änderungen der Summe elektrischen Potentialdifferenzen, welche durch Gewebe, Organ oder Zellsysteme, wie das Nervensystem, erzeugt werden. Biosignale umfassen insbesondere Signale von einem Elektroenzephalogramm (EEG), Elektrokardiogramm (EKG), Elektromyogramm (EMG), Mechanomyogramm (MMG), Elektrookulographie (EOG), Galvanische Hautreaktion (GSR), Elektroenterogramm oder einem Magnetoenzephalogramm (MEG).

Biosignale wie das EEG, EKG, EOG und EMG werden in der Regel mit einem Differenzverstärker gemessen, der die Differenz zwischen zwei an der Haut befestigten Elektroden registriert. Die Biosignale als elektrische Spannungen werden somit stets zwischen zwei Punkten gemessen, die in der Medizin *Ableitungspunkte* genannt werden. Auf diese Punkte werden Elektroden auf die Haut geklebt, die mit einem jeweiligen Gerät (z.B. EEG oder EKG) über elektrische Messkabel verbunden sind. Die gemessenen elektrischen Potentiale werden auch *Ableitungen* genannt.

Die beanspruchte Polymerelektrode sollte mithin zur Ableitung von Biosignalen von einer Haut geeignet. Dies kann eine Ableitung der Biosignale von der Haut eines Menschen oder eine Tieres gleichermaßen. Der Zusatz Polymer-Elektrode meint bevorzugt, dass die Elektrode auf Polymeren basiert und sich als Eigenschaft aus der Verwendung einer lichthärtenden Polymermischung zur Herstellung derselben ergibt.

Vorteilhafterweise ist die Polymerelektrode durch ein überaus einfaches Verfahren in einem Arbeitsgang erhältlich. Hierzu ist lediglich das Auftragen einer lichthärtenden Polymermischung auf ein Substrat (z.B. eine Haut) bevorzugt als dünner Film und eine anschließende Lichthärtung notwendig.

Dünner Film meint bevorzugt, eine Schicht, welche durch das Auftragen der lichthärtenden Polymermischung mit einer Schichtdicke von weniger als 5 mm, bevorzugt weniger als 2 mm, besonders bevorzugt weniger 1mm erhalten wird. Dünn bezeichnet mithin bevorzugt Schichtdicken des Films von weniger als 5 mm, bevorzugt weniger als 2 mm, besonders bevorzugt weniger 1mm. Beispielsweise kann die lichthärtende Polymermischung auf ein Substrat bzw. die Haut mit Schichtdicken von 100 µm bis 2 mm, bevorzugt 500 µm bis 2 mm, besonders bevorzugt 1mm bis 2 mm aufgetragen werden.

Die Querschnittsform des dünnen Films kann unterschiedlich sein, beispielsweise kreisförmig, ellipsenförmig, rechteckig oder quadratisch. Bevorzugt wird der dünne Film jedoch im Wesentlich kreisförmig aufgetragen, sodass kreisförmige Polymerelektroden erhalten werden können.

Die Querschnittsfläche kann ebenfalls an die gewünschte Größe der Elektroden in Abhängigkeit des Anwendungsgebietes angepasst werden. Beispielsweise können Querschnittsflächen von 1 mm² bis 5 cm², bevorzugt 2 mm² bis 2 cm², besonders bevorzugt 5 mm² bis 100 mm², ganz besonders bevorzugt 10 mm² bis 50 mm². Eine Besonderheit der vorliegenden Polymerelektrode, ist es dass diese auch bei sehr kleinen Querschnittsflächen von weniger als 1 cm², bevorzugt weniger als 50 mm², weniger als 25 mm² sehr gute Ergebnisse in Bezug auf die Ableitung von Biosignalen erzielt. Im Gegensatz zu bekannten Elektroden kann durch die Polymerelektroden ein ausgezeichneter Tragekomfort mit höchster Signalqualität vereint werden.

Die Form und Dimensionierung des aufgetragenen dünnen Films der hierin beschriebenen lichthärtenden Polymermischung bestimmt die Form und Dimensionierung der Elektrode.

Die Polymerelektrode wird mithin bevorzugt eine Querschnittsflächen 1 mm² bis 5 cm², bevorzugt 2 mm² bis 2 cm², besonders bevorzugt 5 mm² bis 100 mm², ganz besonders bevorzugt 10 mm² bis 50 mm² und eine Schichtdicke von weniger als 5 mm, bevorzugt weniger als 2 mm, besonders bevorzugt von 100 µm bis 2 mm, bevorzugt 500 µm bis 2 mm oder 1mm bis 2 mm aufweisen.

Zur Härtung der Polymermischung und mithin Fertigstellung einer formstabilen Polymerelektrode erfolgt eine Lichtbestrahlung. Lichtbestrahlung meint bevorzugt die Bestrahlung der Polymermischung, bevorzugt in Form des aufgetragenen dünnen Films, mit einer elektromagnetischen Strahlung. I

Im Sinne der Erfindung soll unter Licht das gesamte elektromagnetische Spektrum vom Ultravioletten Bereich (UV-A bis UV-C: 100 nm bis 380 nm, bevorzugt UV A und UV B: 280 nm bis 380 nm) über den sichtbaren Bereich (380 nm bis 780 nm) bis hin zum Infrarot Bereich (780 nm bis 1mm, bevorzugt near infrared (NIR) 780 nm bis 1.4 µm, shortwavelength infrared (SWIR): 780 nm bis 3 µm) verstanden werden.

Die Lichtbestrahlung kann bevorzugt an die lichthärtenden Komponenten der Polymermischung angepasst sein. Für standardmäßige lichthärtende Zahnzemente auf Dimethyacrlatbasis sind beispielsweise Härtungen mit Blaulichtquellen (440 nm bis 480 nm) bevorzugt.

Nach der Lichtbestrahlung erfolgt durch eine Härtung bzw. Polymerisation eine Aggregatszustandsänderung, sodass die flüssige bzw. gelartige Polymermischung zu einer formstabilen, festen Polymerelektrode wird.

Die Polymerelektrode zeichnet sich dadurch aus, dass diese ohne einen Elektrolyt auskommt, d.h. die elektrophysiologische Ableitung trocken erfolgt. Daher können auch Potentiale im unteren Frequenzbereich (Delta-Band) aufgezeichnet werden. Zudem, ist die Polymerelektrode bzw. deren Vorstufe in Form der Polymermischung aushärtbar. Auch ist die Polymerelektrode nicht polarisierbar und daher geeignet zur Messung von *full band EEGs* (DC). Gegenüber einer lichthärtenden Polymerelektrode des DE-Gebrauchsmusters Nr. 20 2016 003 395, besteht ein deutlich verbessertes Ablöseverhalten von der Haut. Der Widerstand konnte zudem gegenüber der lichthärtenden Polymerelektrode des DE-Gebrauchsmuster DE 20 2016 003 395 verringert werden. Vorteilhaft ist zudem, dass die Polymerelektrode aus einer flüssigen Substanz, beispielsweis einer Tube flexibel applizierbar ist und innerhalb weniger Sekunden mit hoher Präzision ausgehärtet werden kann.

Im Gegensatz zu bekannten Cup-Elektroden werden hierdurch wichtige Voraussetzungen für stabile Langzeitableitungen von Biosignalen erfüllt. Zum einen verliert die Polymerelektrode durch Trocknung nicht ihre Funktionalität, wie es der Fall ist bei der Trocknung gängiger Silber/Silberchlorid Elektroden, welche mit Gel aufgetragen werden. Darüber hinaus ist die Polymerelektrode selbstklebend. Durch die flüssigen Applikation der Polymermischung auf die Haut wird unmittelbar ein adhäsiver Kontakt hergestellt, welcher nach der Härtung aufrechterhalten wird. Ein separater Kleber oder ein Gel zur Erzeugung adhäsiver Kräfte ist vorteilhafterweise nicht notwendig. Zudem ist es möglich die Polymerelektrode auf der Haut unter Haaren aufzubringen, ohne dass eine Rasur erforderlich ist. Vielmehr wird nach der Aushärtung direkt auf der Haut mit blauem Licht eine hochleitfähige und stabile Elektrode erhalten, welche zudem nahezu unsichtbar (transparent) ist.

Durch diese Eigenschaften ist die Elektrode ideal für praxisnahe Anwendungen, z.B. bei der Epilepsiediagnostik oder der Schlaganfallprävention. Die flache, glatte Beschaffenheit und besonders kleine Dimensionierung machen die Polymerelektroden für den Anwender äußerst angenehm. Dies ist wichtig, um beispielsweise Druckstellen bei der Schlafdiagnostik oder bei einer Applikation an Kleinkindern bzw. Neugeborenen zu vermeiden.

Weiterhin kann die Elektrode durch Druck von zwei Seiten überaus leicht entfernt werden. Mechanische Messungen mit dem Elektrodenmaterial zeigen äußert geringe Kräfte, welche für das Reißen des Materials erforderlich sind. Diese Eigenschaft sorgt zuverlässig für eine Vermeidung von Hautverletzungen und vermindert das Risiko des Auftretens von Schmerzen bei der Entfernung der Elektroden. Die Elektrode kann auch mit Hilfe von medizinischem Ultraschall Gel entfernt werden.

In einer bevorzugten Ausführungsform erfolgt die Härtung mittels einer Lichtbestrahlung im Blaubereich. Im Sinne der Erfindung meint Blaubereich bevorzugt eine Lichtbestrahlung im sichtbaren Spektralbereich, welche gemeinhin der Farbe Blau zugeordnet wird. Bevorzugt meint Blaubereich eine Lichtbestrahlung in einem Wellenlängenbereich von 440 nm bis 480 nm. Für standardmäßige lichthärtende Zahnzemente werden Härtungen bevorzugt mit Blaulichtquellen im Bereich von 440 nm bis 480 nm durchgeführt. Geeignete Blaulichtquellen sind daher vielfältig kommerziell erhältlich. Zudem sind für Blaulichtquellen im Gegensatz zu beispielweise UV- oder Infrarotlichtquellen keine erhöhten Sicherheitsmaßnahmen notwendig, sodass eine unkomplizierte Implementation in den Klinikalltag möglich ist.

In einer bevorzugten Ausführungsform erfolgt die Härtung mittels einer Lichtbestrahlung über einen Zeitraum von 5 - 60 Sekunden, bevorzugt von 10 - 20 Sekunden. Vorteilhafterweise reichen für die erfindungsgemäßen Polymermischung bzw. bevorzugten Ausführungsformen überaus kurze Bestrahlungszeiten, um eine hinreichende Aushärtung zu gewährleisten. Auch die Bereitstellung von Ableitungen mit einer hohen Anzahl an Elektroden, wie Sie beispielsweise für medizinische Montagen bei Frühgeborenen empfohlen wird, ist somit schnell und zuverlässig möglich.

In einer bevorzugten Ausführungsform weist die Polymerelektrode eine Impedanz von weniger gleich 10 kOhm, bevorzugt weniger gleich 4 kOhm, besonders bevorzugt von weniger gleich 2 kOhm auf. Bevorzugt werden die vorgenannten niedrigen Impedanzen über einen Frequenzbereich von 1 Hz bis 10 000 Hz realisiert.

Unter der Impedanz wird bevorzugt im üblichen Sinne ein Wechselstromwiderstand verstanden, welcher das Verhältnis von elektrischer Spannung zur Stromstärke bevorzugt bei zweipoligen Netzwerkelementen angibt. Beispielsweise kann die Impedanz einer Polymerelektrode auf der Haut gegen eine zweite Polymerelektrode in einem Abstand von 10 cm bestimmt werden. Zur Bestimmung der ggf. frequenzabhängigen Impedanz der Polymerelektroden können bekannte und kommerzielle erhältliche Impedanzmessgeräte verwandt werden. Beispielweise eignet sich das *Impedance analyser module* des Stem lab, Solkan, Slovenia oder auch der *Impedance Analyser* LCR-6100 der Firma GW Instek, Taiwan.

Die geringe Impedanz über einen weiten Frequenzbereich, insbesondere auch bei niedrigen Frequenzen von 10 Hz bis 100 Hz, stellt einen wichtigen Vorteil für eine umfassende spektrale Aufzeichnung von Biosignalen dar.

Zudem zeigt die Polymer Elektrode eine sehr gute DC (Gleichstrom) Drift Charakteristik, analog zu die gesinterten Ag/AgCl Elektroden, bei full band DC EEG Messungen. Dies macht die Elektrode besonders interessant für Anwendungen auf der neonatologischen Intensivstation, da die langsamen Wellen des EEGs (beispielsweise Delta-Wellen) eine besondere Rolle bei der Hirnreifung spielen und daher bei der Überwachung von Frühgeboren hohe Relevanz zukommt.

Beispielsweise kann eine Ableitung des vollen Spektrums der EEG Wellen (*full band EEG*) erfolgen, welche auch langsamere Delta-Schwingungen Frühgeborener umfasst.

In einer bevorzugten Ausführungsform der Erfindung ist die Polymerelektrode dadurch gekennzeichnet, dass innerhalb der Polymerelektrode ein oder mehrere leitfähige Fasern eingebracht vorliegen.

In einer bevorzugten Ausführungsform der Erfindung ist die Polymerelektrode dadurch gekennzeichnet, dass diese herstellbar ist durch ein Verfahren umfassend die Schritte
a. Bereitstellung einer leifähigen erfindungsgemäßen Polymermischung oder einer bevorzugten Ausführungsform hiervon
b. Bereitstellung einer oder mehrerer leitfähiger Fasern
c. Auftragen der leifähigen Polymermischung als dünnen Film auf die Haut
d. Einbringen des leitfähigen Fasern in die lichthärtende Polymermischung
e. Härtung der leifähigen Polymermischung mittels einer Lichtbestrahlung

Im Sinne der Erfindung meint eine leitfähige Faser eine lineares Gebilde, welche auf einem Faserstoff basiert, beispielsweise Naturfasern oder Kunststofffasern. Die Faser ist insbesondere im Verhältnis zu ihrer Länge ein dünnes, flexibles Gebilde. Beispielsweise kann das Verhältnis von Länge zu Durchmesser mindestens zwischen 3:1 und 10:1 oder mehr liegen. Mechanisch sind Fasern vorzugsweise flexibel, d.h. sie können in Längsrichtung keinen Druck-, sondern nur Zugkräfte aufnehmen. Bei einer Druckbelastung werden die Fasern sich vielmehr beugen bzw. knicken.

In die Polymerelektrode können sowohl einzelne Fasern als auch ein Verbund von mehreren Fasern (Garn) eingebracht werden. Beispielsweise kann es bevorzugt sein, mehrere Fasern zu einem Garn zu flechten oder zu verdrillen. Bei den Fasern kann es sich sowohl um Naturfasern handeln, als auch um Kunststofffasern, wobei mit Kunststofffasern insbesondere mit Kunststofffasern auf Basis von Polyamid oder Polyester besonders gute Ergebnisse erzielt werden können.

Die Leitfähigkeit der Fasern, bevorzugt Kunststofffasern, kann durch ein Einziehen bzw. Eindringen einer PEDOT-Verbindung, bevorzugt PEDOT-PSS, erzielt werden. Beispielsweise kann ein Kunststoffgarn in einer Dispersion umfassend PEDOT-PSS gegeben bzw. getränkt werden. Durch passive Diffusion oder Kapillarkräfte dringt das PEDOT-PSS in das Kunststoffgarn ein, sodass eine leitfähige Faser bzw. ein leitfähiges Fasergarn erhalten wird. Besonders gute Ergebnisse werden beispielsweise mit einem Sacknähgarn aus Kunststofffasern erzielt, welche über Nacht bei 35°C in einem Bad aus PEDOT PSS (z.B. 200ml) und Menthol (z.B. 2 mg) gelagert wird. Auch mehrmalige Tränkungen für kürzere Zeiträume können bevorzugt sein. Die leitfähigen Fasern werden bevorzugt im Anschluss getrocknet z.B. im Trockenschrank oder bei Raumtemperatur.

Anschließend kann es bevorzugt sein die leitfähigen Fasern bzw. das Fasergarn bis auf endseitige Kontaktstellen, mit einer elektrisch isolierenden Schicht, z.B. einem isolierenden Silikon zu überziehen. Das erhaltene Kabel kann im Sinne der Erfindung auch als Textilkabel bezeichnet werden, da es im Wesentlichen aus Fasern besteht, welche als Textilfasern auch zur Herstellung von Textilien verwandt werden können. Mittels des PEDOT wird den Textilfasern eine Leitfähigkeit vermittelt, während die Isolierschicht eine Nutzung als Kabel erlaubt.

Für eine Kontaktierung an ein Messgerät zur Erfassung von Biosignalen kann an das eine Ende des Textilkabels bzw. der leitfähigen Faser ein Stecker gecrimpt werden. Das andere Ende des Textilkabels bzw. der leitfähigen Fasern bleibt frei und wird direkt in die noch flüssige Polymermischung gegeben, bis diese durch Lichtbestrahlung ausgehärtet ist und das Textilkabel hält. Bevorzugt enthält das Textilkabeln bzw. die leitfähigen Fasern kein Metall.

Im Gegensatz zu klassischen Leitungskabeln, welche in der Regel einen Metalldraht sowie eine Isolierschicht umfassen, zeichnen sich die leitfähigen Fasern durch eine hohe Flexibilität aus. Insbesondere für die Ableitung von ggf. bewegenden Probanden oder Patienten beispielsweise Kindern oder Tiere stellt dies einen wesentlichen Vorteil dar, welche die Anbringung und Handhabung Polymerelektroden erleichtert. Die Ausführungsform eignet sich somit zudem insbesondere für Anwendungen zum Langzeitmonitoring oder besonderen Belastungen, z.B. bei frei laufenden Tieren. In einigen Ausführungsformen kann es hierfür zudem bevorzugt sein, die Polymerelektroden zusätzlich zu schützen, beispielsweise mittels eines Körpersilikons (z.B. Body Double SILK).

Zudem interferieren die faserbasierten Kabel vorteilhafterweise nicht stark mit externen elektromagnetischen Feldern. Eine Verwendung der leitfähigen Fasern zur Ableitung elektrischer Biosignale kann daher besonders bevorzugt für Ableitungen (z.B. EKG, EEG) im Rahmen einer MRT-Untersuchung erfolgen.

In einem weiteren Aspekt betrifft die Erfindung eine Verwendung einer hierin beschriebenen Polymerelektrode für die Ableitung elektrischer Biosignale, bevorzugt für die Elektroenzephalografie (EEG), die Elektrokardiographie (EKG) und/oder die Elektromyografie (EMG).

Die Elektroenzephalografie (EEG) meint bevorzugt im üblichen Sinne eine Methode der medizinischen Diagnostik und der neurologischen Forschung zur Messung der summierten elektrischen Aktivität des Gehirns durch Aufzeichnung der Spannungsschwankungen an der Kopfoberfläche. Da die auf der Kopfhaut zu messenden Signale in der Größenordnung von 5 bis 100 µV liegen, werden empfindlicher Messgeräte bzw. Verstärker benötigt. Zudem hängt die Signalqualität von den verwandten Elektroden ab, wobei sich die vorliegenden Polymerelektroden aufgrund ihrer niederohmigen Eigenschaften und der engen Hauthaftung als besonders geeignet erwiesen haben.

Ein Elektrokardiogramm (EKG) meint bevorzugt im üblichen Sinne die Aufzeichnung der Summe der elektrischen Aktivitäten aller Herzmuskelfasern mittels der Elektroden zur Ableitung der elektrischen Biosignal und eines Elektrokardiografen.

Die Elektromyografie (EMG) bezeichnet bevorzugt im üblichen Sinne eine elektrophysiologische Methode in der neurologischen Diagnostik, bei der die elektrische Muskelaktivität einzelner motorischer Einheiten gemessen werden.

In einer weiteren bevorzugten Ausführungsform betrifft die Erfindung die Verwendung einer hierin beschriebenen Polymerelektrode zur Ableitung der elektrischen Biosignale, wobei die Ableitung während einer Magnetresonanztomographie-Untersuchung erfolgt.

Unter der Magnetresonanztomographie wird bevorzugt jegliches bildgebendes Verfahren verstanden, welches auf Prinzipien der Kernspinresonanz (englisch *Nuclear Magnetic Resonance,* NMR) basiert und durch Erzeugung von Schnittbilder in der medizinischen Diagnostik verwandt wird, um Strukturen oder Funktionen von Geweben und Organen darzustellen. Synonym Begriffe sind die Kernspintomographie oder *Magnetic Resonance Imaging* (MRI). Zur Durchführung einer MRT-Untersuchung werden Magnetresonanztomographen verwandt, welche wechselnde Magnetfelder mit teilweise hohen Feldstärken generieren können.

Um die Bildgebung der Magnetresonanztomographen zu steuern, wird häufig parallel eine Ableitung eines EKGs vorgenommen. Die Elektroden sowie die ableitenden Kabel liegen mithin während Untersuchung ebenfalls im Tomographen vor. Um eine Interferenz mit der Bildgebung zu vermeiden, sollten die Elektroden sowie die damit verbundenen Kabel keine ferromagnetischen Materialien aufweisen. Teilweise ist es im Stand der Technik bekannt aus diesem Grund karbonbasierte Elektroden und Kabeln zu verwenden. Deren Handhabung ist aufgrund der hohen Steifigkeit der Materialien jedoch aufwendig und störanfällig. Die vorliegenden Polymerelektroden, insbesondere bei gleichzeitiger Verwendung leitfähiger Textilkabel bzw. leitfähiger Fasern vermeiden nicht nur eine Störung der MRT-Bildgebung, sondern zeichnet sich zudem durch eine besonders unkomplizierte Handhabung aus. Das Auftreten von Fehlern bei der EKG Aufzeichnung wird somit reduziert.

In einem weiteren Aspekt betrifft die Erfindung zudem eine Vorrichtung zur Applikation einer erfindungsgemäßen Polymerelektrode auf einer Haut umfassend
- einen Spritzenkörper mit einem Spritzenauslauf und einem Spritzenbehälter eingerichtet zur Aufnahme einer leifähigen Polymermischung
- einen Kolbenaufsatz mit einem Spritzenkolben, welcher für die Aufnahme in den Spritzenbehälter und den Ausstoß der lichthärtenden Polymermischung durch den Spritzenauslauf konfiguriert ist
wobei eine oder mehrere Lichtquellen ringförmig um den Spritzenauslauf angeordnet vorliegen und eine Steuereinheit auf Basis eine Auslösesignales eine Aktivierung der Lichtquellen für eine Beleuchtungszeit steuert.

Die genannte Vorrichtung zeichnet sich dadurch aus, dass mittels dieser Vorrichtung eine Applikation der Polymerelektrode auf einer Haut auf besonders einfach und zuverlässiger Weise erzielt werden kann. Die Vorrichtung umfasst zu diesem Zweck einen Spritzenkörper mit einem Spritzenauslauf und einem Spritzenbehälter eingerichtet zur Aufnahme einer leifähigen Polymermischung oder bevorzugte Ausführungsformen hiervon.

Der Spritzenbehälter ist bevorzugt ein Hohlkörper, der zur Aufnahme und zum Halten einer flüssigen Substanz, wie beispielsweise einer Polymermischung, geeignet ist. Verschiedene Materialien können für den Behälter verwandt werden. Vorzugsweise ist das Material flüssigkeitsdicht. Beispiele für geeignete Materialien sind Glas, Kunststoff, vorzugsweise Hartkunststoff oder andere dauerhafte Materialien.

In einer Ausführungsform ist das Material transparent, so dass die Farbe und/oder der Füllstatus der Polymermischung sichtbar ist. In einer weiteren Ausführungsform ist das Material für den Spritzenbehälter zumindest teilweise nicht transparent für Licht einer Wellenlänge, welche für die Aktivierung der Polymermischung vorgesehen ist. Beispielsweise kann das Material für den Spritzenbehälter für blaues Licht intransparent sein, während rotes Licht oder Licht einer anderen Farbe passieren kann. Hierdurch können besonders gute Lagerungsbedingungen erzielt werden. Die Form des Behälters kann beispielsweise konisch, quaderförmig oder zylindrisch sein. Bevorzugten Volumina oder Fassungsvermögen liegen zwischen 0,1 ml und 100 ml, vorzugsweise 0,5 ml und 20 ml.

Wie hierin verwendet, bezieht sich der Spritzenauslauf auf den stromabwärts gelegenen Extrusionsbereich der Vorrichtung. An dem abgebenden Ende weist der Spritzenauslauf typischerweise eine wesentlich geringere Querschnittsfläche auf, als am stromaufwärts gelegenen Ende, um eine genaue Abscheidung der Polymermischung zu ermöglichen. Der Spritzenauslauf kann durch eine zumindest teilweise konische Form gekennzeichnet sein und in bevorzugter Ausführungsform eine stumpfe Nadel, eine Spitze oder Düse umfassen. Der Spritzenauslauf kann aus demselben oder einem unterschiedlichen Material wie der Spritzenbehälter gefertigt sein. Auch ist es möglich, dass Spritzenauslauf und Spritzenbehälter monolithisch verbunden sind oder aber zusammengesetzt werden können.

Der Kolbenaufsatz umfasst einen Spritzenkolben, welcher dafür eingerichtet ist, um im Spritzenbehälter aufgenommen zu werden und eine Polymermischung aus dem Behälter und durch den Spritzenauslauf zu extrudieren. Vorzugsweise umfasst der Spritzenkolben zu diesem Zweck einen Stopfen. Der Stopfen ist vorzugsweise so bemessen, dass die äußere Querschnittsfläche des Stopfens der inneren Querschnittsfläche des Behälters entspricht, was zu einer (material- bzw. flüssigkeitsdichten) Trennung eines ersten Volumens im Behälter stromabwärts eines Stopfens und eines zweiten Volumens des Behälters stromaufwärts des Stopfens führt.

Durch Bewegen des Kolbens, an dem der Stopfen befestigt ist, wird die im ersten Volumen befindliche Polymermischung in den nachgeschalteten Spritzenauslauf geleitet. Kolben und Stopfen können aus verschiedenen geeigneten Materialien hergestellt werden, beispielsweise Kunststoff (biegsam oder unbiegsam) Glas und/oder Gummi. In bevorzugten Ausführungsformen sind Kolben, Stopfen sowie Behälter inerte Materialien, ohne Einfluss auf eine Reaktion der Polymermischung. Bevorzugt stellt der Kolbenaufsatz eine separate Baukomponente dar, welche in den Spritzenbehälter, beispielsweise nach dessen Befüllung mit einer Polymermischung, eingebracht werden kann.

Die Vorrichtung ist dadurch gekennzeichnet, dass um den Spritzenauslauf ein oder mehrere Lichtquellen angeordnet sind. Die Anordnung der Lichtquellen orientiert sich bevorzugt an der Geometrie des Spritzenauslaufes. Ist beispielsweise der Spritzenauslauf konisch oder weist einen kreisförmigen Querschnitt auf, werden die Lichtquellen bevorzugt ringförmig um selbigen angeordnet. Bevorzugt umfasst die Vorrichtung zwei, drei, vier oder mehr Lichtquellen, welche entlang des Umfanges des Spritzenauslaufes, beispielsweise ringförmig, angeordnet vorliegen.

Die Lichtquellen werden von einer Steuereinheit auf Basis eines Auslösesignal automatisch aktiviert und für eine bevorzugt vorbestimmte Beleuchtungszeit gesteuert. Besonders bevorzugte Lichtquellen sind LEDs.

Im Sinne der Erfindung ist die Steuereinheit bevorzugt eine elektrische Schaltung, ein Prozessor, ein Prozessorchip, Mikroprozessor, ein Mikrokontroller oder ein integrierter Schaltkreis, welcher konfiguriert ist, um elektrischen Komponenten der Vorrichtung, insbesondere die Lichtquellen, zu steuern. In einer bevorzugten Ausführungsform umfasst die Steuereinheit einen Mikroprozessor.

Im Sinne der Erfindung wird unter einem Mikroprozessor bevorzugt eine Datenverarbeitungsvorrichtung, d.h. ein Prozessor, verstanden, welcher sich durch kleine Abmaße im Bereich von einigen mm auszeichnet und wobei bevorzugt alle Bausteine des Prozessors auf einem Mikrochip oder auch integriertem Schaltkreis (engl. integrated circuit, IC) vorliegen. Der Mikroprozessor kann bevorzugt auch ein Mikrokontroller sein, welcher neben dem Prozessor weitere periphere Elemente auf dem Mikrochip integriert und beispielsweise auch über einen Datenspeicher verfügt. Es ist weiterhin bevorzugt, dass der Mikroprozessor auf einer Leiterplatte (engl. *printed circuit board, PCB*) installiert vorliegt.

Die Steuereinheit ist bevorzugt dazu eingerichtet auf Basis eines Auslösesignal die Lichtquellen für eine vorbestimmte Zeit zu aktivieren. Bei dem Auslösesignal kann es sich um ein elektrisches Signal handeln, welches durch manuelle Betätigung eine Bedienelementes, beispielsweise eine Bedienknopf oder Tastschalters, generiert wird.

Es kann jedoch auch bevorzugt sein, dass das Auslösesignal auf Basis einer Detektion der Betätigung des Kolbens und mithin einer Extrusion der Polymermischung generiert wird.

In jedem Fall wird durch das Auslösesignal zuverlässig für eine bevorzugt vorbestimmte Zeit eine Aktivierung der Lichtquellen und somit eine Lichtbestrahlung vollzogen.

Durch die vorgesehene Anordnung der Lichtquellen unmittelbar um den Spritzenauslauf kann eine Lichthärtung erreicht werden, welche zeitlich optimal mit dem Auftragen der Polymermischung auf die Haut korreliert.

Zum einen wird durch die gleichzeitige Lichtbestrahlung während des Ausstoßprozesses bereits im Spritzenauslauf eine Polymerisationsreaktion initiiert, wodurch eine besonders stabile Applikation und Formgebung der Polymerelektroden erreicht wird. Zudem ist die Handhabung unkompliziert mit einer Hand möglich und bedarf keiner weiteren separaten Geräte, um die Lichtbestrahlung zu durchzuführen. Hervorzuheben ist weiterhin, dass durch die Integration der Lichtquellen in den Spritzenauslauf auf konstruktiv einfache Weise reproduzierbare Bedingungen der Lichthärtung sichergestellt werden können, welche zuverlässig zu gleichmäßigen und hochqualitativen Polymerelektroden führen.

Eine derartige Vorrichtung, welche optimal zur Applikation einer Polymermischung geeignet ist, wird weder durch den Stand der Technik vorbeschrieben, noch durch diesen nahegelegt. Stattdessen werden im Stand der Technik separate Systeme für das Auftragen und eine Härtung von lichthärtenden Substanzen beschrieben.

Beispielsweise werden viele Subtanzen in Spritzen gefüllt vertrieben und müssen für die jeweilige Anwendung in einzelne Portionen aufgeteilt und verwendet werden. Dies betrifft z.B. Medikamente, Klebstoffe oder Wärmeleitpasten. Für diese Anwendungen existieren auf dem Markt verschiedene Systeme. Hierzu zählen Spritzen mit Gewindekolben, bei denen der Hub und damit die abgegebene Menge mittels einer Sperre, meist einem Rändelrad, eingestellt werden kann. Aufsätze in Stab- oder in Pistolenform, bei denen man den Hub teil mechanisch, teils elektronisch vorgeben kann. Auch spezielle Systeme, z.B. in Form eines Pens für die Insulinapplikation sind bekannt.

Für die Aushärtung von lichthärtenden Substanzen sind separate Vorrichtungen. Im größten Einsatzbereich für lichthärtende Polymere, der Zahnbehandlung, erfolgt dies zumeist mittels akkubetriebener Handgeräte, die eine blaue Hochleistungs-LED enthalten und mittels Lichtleiter das blaue Licht bündeln. Bei einem weiteren großen Anwendungsbereich, der Nagelbehandlung, werden zumeist kleine Kammern mit UV-Licht eingesetzt, bei denen die Finger vollständig in die Kammer eingeführt werden. Auch bei lichthärtenden Klebstoffen, werden zumeist blaue LEDs verwandt. Hier ist allerdings in einigen Fällen bereits die blaue LED bereits der Vorrichtung integriert. Nach dem Applizieren des Klebstoffs wird der Behälter gedreht und die blaue LED eingeschaltet. Auch bei diesen Geräten erfolgt jedoch keine homogene Lichthärtung im Extrusionsbereich. Stattdessen erfolgt in einem separaten Schritt eine Beleuchtung mit einer LED, welche lediglich bereits an der Vorrichtung installiert vorliegt.

Im Gegensatz zu einer Lichtbestrahlung mit separaten Lichtquellen wird durch eine Integration der Lichtquellen in den Spritzauslauf vermieden, dass durch unterschiedliche Bestrahlungsabstände, welche bei einer manuellen Handhabung nicht zu vermeiden sind, stets gleiche Bedingungen sichergestellt werden. Die hierdurch erreichten reproduzierbaren Bedingungen bei der Lichthärtung führen zu ausgezeichneten homogenen Polymerelektroden, welche in Bezug auf die Ableitung elektrischer Biosignale zuverlässige, aussagekräftige Ergebnissen sicherstellen.

Der Fachmann erkennt, dass bevorzugte Ausführungsformen und Vorteile, welche mit im Zusammenhang mit der erfindungsgemäßen Polymermischung bzw. den daraus herstellbar Polymerelektroden offenbart wurden, sich gleichermaßen auf die erfindungsgemäße Vorrichtung zur Applikation einer Polymerelektrode gelten übertragen.

In einer bevorzugten Ausführungsform sind die Lichtquellen daher Blaulichtquellen mit einer Emissionswellenlänge von 440 nm bis 480 nm. Derartige Blaulichtquellen sind besonders gut geeignet, um eine Polymermischung mit einem lichthärtenden auf Dimethacrylaten basierenden Zahnzement auszuhärten.

In einer bevorzugten Ausführungsform der Vorrichtung liegen die eine oder mehrere Lichtquellen sowie die Steuereinheit in einem Bestrahlungsaufsatz integriert vor, welcher auf den Spritzkörper lösbar installiert werden kann. Bevorzugt kann in dem Bestrahlungsaufsatz zudem eine Energiequelle, beispielsweise eine Batterie, zum Betreiben der Lichtquellen integriert vorliegen. In der Ausführungsform umfasst die Vorrichtung mithin bevorzugt mindestens zwei voneinander lösbare Komponenten, einen Bestrahlungsaufsatz sowie einen Spritzenkörper. Besonders bevorzugt ist auch der Kolbenaufsatz lösbar mit dem Spritzkörper verbunden bzw. kann auf lösbare Weise in den Spritzenbehälter eingebracht werden.

Vorteilhafterweise kann bei dieser Ausführungsform sowohl der Bestrahlungsaufsatz, als auch der Kolbenaufsatz für mehrere Anwendung wiederverwendet werden, während beispielsweise der Spritzenkörper mit der befüllten Polymermischung ausgetauscht wird.

Dies erlaubt eine besonders kosteneffektive und wirtschaftliche Nutzung der Vorrichtung. Komponenten, welche aufwendiger in der Produktion sind, können wiederverwendet werden können. Einfache Komponenten, welche günstig in der Herstellung sind, jedoch verschmutzen, sind vorteilhafterweise austauschbar.

Beispielsweise kann der Spritzenkörper vorgefertigt mit einer Polymermischung bereitgestellt werden kann. Zur Applikation der Polymerelektrode wird anschließend lediglich der Bestrahlungsaufsatz sowie der Kolbenaufsatz bereitgestellt. Ein Zusammensetzen der Komponenten zu einer Vorrichtung mit welcher sich die Polymerelektrode applizieren lässt, kann unmittelbar vor der ersten Anwendung erfolgt. Nach mehrmaliger Applikation von Polymerelektroden und vollständiger Entleerung des Spritzenkörpers, kann dieser gegen einen Neuen bereits mit einer Polymermischung vorbeladenen Spritzenkörper ausgetauscht werden.

Der Bestrahlungsaufsatz wird bevorzugt eine Form aufweisen, welche eine lösbare Arretierung an den Spritzenkörper ermöglicht. Beispielsweise kann es bevorzugt sein, dass der Strahlungsaufsatz zwei Halterungen aufweist, welche kongruent zum Spritzenauslauf bzw. Spritzenkörper ausgelegt sind. So kann die ersten Halterung beispielsweise einen inneren (Ring-)Durchmesser aufweist, welcher dem äußeren (Ring-)Durchmesser des Spritzauslaufes entspricht und die zweite Halterung einen inneren (Ring-)Durchmesser aufweisen, welche dem äußeren (Ring-)Durchmesser des Spritzenbehälters entspricht. Vorzugsweise liegt in der ersten Halterung eine oder mehrere Lichtquelle integriert vor, welcher sich im montierten Zustand unmittelbar um den Spritzenauslauf fügen. Die Halterungen können bevorzugt durch einen Steg verbunden sein, welche beispielsweise die Steuereinheit beinhaltet.

Bei entsprechender Dimensionierung kann die lösbare Verbindung lediglich durch den adhäsiven Kontakt der Befestigungshalterungen mit dem Spritzenkörper hergestellt werden. Es kann aber auch bevorzugt sein, zusätzliche Verschluss- oder Rastmechanismen bereitzustellen.

In einer weiteren bevorzugten Ausführungsform umfasst die Vorrichtung einen Sensor zur Detektion einer Bewegung des Spritzkolbens, wobei das Auslösesignal zur Aktivierung der Blaulichtquellen einer detektierten Bewegung des Spritzkolbens entspricht.

Verschiedenen Sensoren können zu diesem Zweck eingesetzt werden. Beispielsweise können induktive oder kapazitative Näherungssensoren verwandt werden. Diese können beispielsweise den Abstand zwischen einer Position auf dem Spritzkolben und einer Position auf dem Spritzbehälter bestimmen und somit eine relative Bewegung erfassen. In einer bevorzugten Ausführungsform liegt am vorderen Ende des Spritzkolbens eine Magnet, bevorzugt ein Neodynmagnet vor, dessen Magnetfeld von einem Detektor erfasst werden kann, welcher bevorzugt ortsfest mit dem Spritzenkörper verbunden ist.

Die automatisierte Aktivierung der Lichtquellen anhand der Bewegung des Kolbens führt zu einer optimierten zeitlichen Synchronisation des Ausstoßes und der Lichthärtung und somit besonders zuverlässig zu hochqualitativen Polymerelektroden.

In einer bevorzugten Ausführungsform beträgt die Beleuchtungszeit zwischen 5-60 Sekunden, bevorzugt zwischen 10-20 Sekunden. Die Beleuchtungszeit meint bevorzugt jenen Zeitraum über welchen die Lichtquellen mittels der Steuereinheit für eine Emission aktiviert werden. Für die vorgenannten Parameter konnten für die erfindungsgemäße Polymermischung ausgezeichnete Härtungsergebnisse erzielt werden.

In einer bevorzugten Ausführungsform umfasst die Vorrichtung Positioniermittel zur Einbringung einer oder mehrere leitfähiger Fasern, bevorzugt einer oder mehrerer leitfähiger Kunststofffasern in die Polymerelektrode.

Bei den Positioniermitteln zur Einbringung der leitfähigen Fasern kann es sich bevorzugt um eine Öse handeln, durch welche die leitfähigen Fasern bzw. das Textilkabel in Bezug auf den Spritzenauslauf positioniert werden. So kann es bevorzugt sein, die leitfähigen Fasern beispielsweise in Form eines Textilkabels, bei welchem die leitfähigen Fasern mit einer Isolierschicht ummantelt sind, in die Öse einzuführen, wobei das vordere Ende des Textilkabels bevorzugt freiliegende leitfähiger Fasern aufweist und nahe der Öffnung des Spritzenauslauf fixiert vorliegt.

Dies erlaubt eine besonders einfache Handhabung der Vorrichtung zur Bereitstellung einer Polymerelektrode mit bereits integriertem Ableitungskabel, vorzugsweise umfassend ein oder mehreren leitfähigen Fasern. Durch Integration der Fixierung eines Ableitkabels kann eine umständliche beidhändige Handhabung vermieden. Auch Fehler bei der Einbringung eines Textilkabels bzw. der leitfähigen Fasern werden mittels der Positioniermittel signifikant reduziert.

In einer weiteren bevorzugten Ausführungsform weist die Vorrichtung zudem ein Silikonplättchen auf, welches bevorzugt endseitig um den Spritzenauslauf angeordnet ist und für die Polymerelektrode als Formgeber bzw. Abstandshalter fungiert. Beim Auftragen breitet sich die Polymermischung zwischen dem Substrat und dem Silikonplättchen aus und stellt somit die Ausbildung eines dünnen Films der Polymerelektroden sicher. Beispielsweise kann durch ein kreisförmiges Silikonplättchen bevorzugt eine kreisförmige, dünnschichtige Polymerelektrode erhalten werden.

In einer bevorzugten Ausführungsform ist die Vorrichtung dadurch gekennzeichnet, dass der Kolbenaufsatz eine Dosiereinheit bzw. einen Dosiermechanismus umfasst, welches die Abgabe einer vorbestimmten Menge an einer Polymermischung bei einmaliger Betätigung der Dosiereinheit sicherstellt.

Im Stand der Technik sind hierzu verschiedenen Systeme bekannt beispielsweise können spiralförmige Spritzkolben verwandt werden, wobei der Kolbenaufsatz derart konfiguriert ist, das bei einmaliger Betätigung eines Bedien- oder Druckknopfes lediglich eine Rotation um einen vorbestimmten Winkel vorgesehen ist, welche einer vorbestimmten Translation des Kolbens und mithin einer vorbestimmte Dosiermenge der Polymermischung entspricht.

In einer bevorzugten Ausführungsform ist die Vorrichtung dadurch gekennzeichnet, dass der Kolbenaufsatz eine Aufsatzhülse zur Führung des Spritzkolbens, einen Druckknopf zur Steuerung des Spritzkolbens und eine Rückstellfeder für den Spritzkolben umfasst.

Besonders bevorzugt ist, dass der Spritzkolben eine Kolbenstange mit einer Zahnung aufweist, wobei die Zahnung in Bezug auf den Umfang der Kolbenstange bevorzugt in zwei gegenüberliegenden Bereichen vorliegt, welche von einem Bereich ohne Zahnung getrennt werden.

Weiterhin ist es bevorzugt, dass der Druckknopf zwei Zangenpaare umfasst, wobei ein Führungszangenpaar der Führung des Druckknopfes in der Aufsatzhülse und eine Steuerungszangenpaar zur Steuerung des Spritzkolbens dient.

Die Ausführungsformen der Vorrichtung erlauben mittels konstruktiv einfacher Mitteln eine besonders robuste Dosierung einer vorbestimmten Menge der Polymermischung.

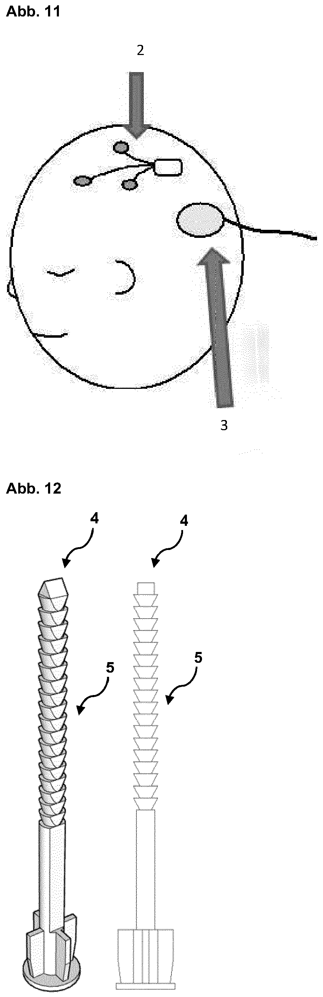

Bevorzugt besitzt der Spritzenkolben hierzu eine Kolbenstange mit einer Zahnung, wobei die Zahnung in Bezug auf den Umfang der Kolbenstange bevorzugt in zwei gegenüberliegenden Bereichen vorliegt, welche von einem Bereich ohne Zahnung getrennt werden. Eine beispielhafte Ausführungsform ist in Abb. 12 dargestellt ist. Bevorzugt können die Bereiche mit bzw. ohne Zahnung jeweils abwechselnd ein Viertel (90°) des Umfanges einnehmen.

Die Zahnung ermöglicht es einem im Druckkopf integrierten Zangenpaar, den Kolben entweder zu bewegen, oder aber über die Zacken (der Zahnung) hinwegzugleiten. Die Verhaltensweise hängt von der Winkelstellung der Kolbenstange oder des Zangenpaares ab und ändert sich jeweils mit jeder 90 Grad Drehung. Der Abstand der Zacken (der Zahnung) bestimmt dabei den Vorschub und über das Spritzenvolumen die abgegebene Dosiermenge. Am Ende der Kolbenstange befindet sich bevorzugt eine Spitze, die ein sicheres Einfädeln in das Zangenpaar ermöglicht.

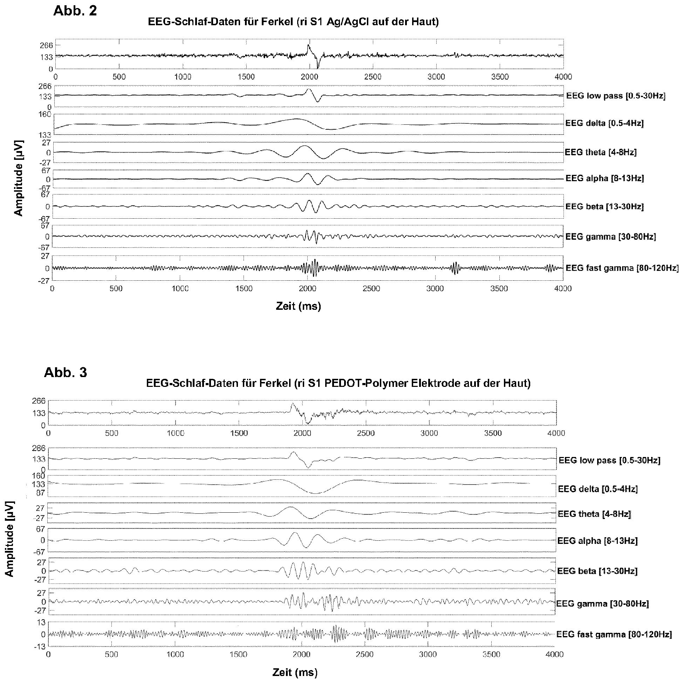

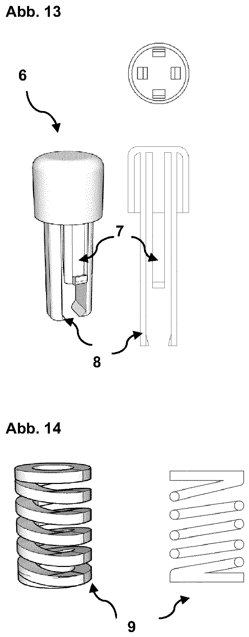

Der Druckknopf besteht bevorzugt zum einen aus einem Knopf zum Drücken, zum anderen aus zwei Zangenpaaren. Ein beispielhafter Druckknopf ist in (Abb. 2) dargestellt. Das eine Zangenpaar (Steuerzangen, vgl. Abb. 13B) steuert bevorzugt die Kolbenstange, das andere Zangenpaar (Führungszangen, vgl. Abb. 13A) dient bevorzugt zur Fixierung des Knopfes in der Aufsatzhülse. Dazu besitzen diese Zangen bevorzugt Haken, welche gegen die Innenfläche der Aufsatzhülse gerichtet sind. Eine Drehung des Zangenpaars kann erfolgen, indem man den Knopf dreht.

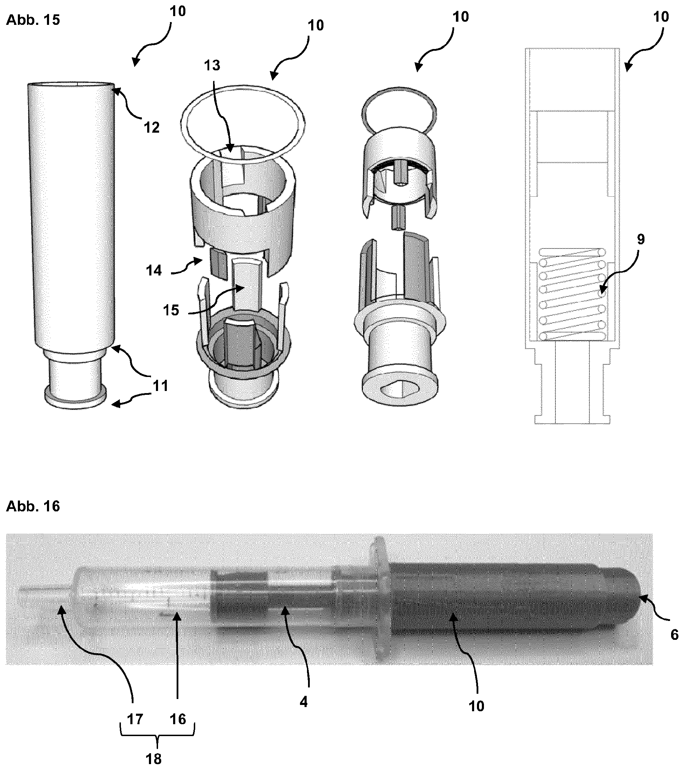

Die Aufsatzhülse (für eine beispielhafte Ausführungsform vgl. Abb. 15) besitzt bevorzugt am unteren Teil zwei Passungen, die zum Fixieren der Aufsatzhülse im Spritzenkörper bzw. Spritzenbehälter dienen (siehe auch Abb. 15A). Im oberen Teil befindet sich bevorzugt eine Passung für eine leichtgängige Führung des Druckknopfes (siehe auch Abb. 15B). Als Anschlag dient bevorzugt eine Kante, die bei der Verjüngung des Innendurchmessers entsteht. Die Steuerung und Fixierung des Druckknopfes erfolgen durch Konturen in der Innenfläche der Aufsatzhülse. Zur Erleichterung der Montage des Druckknopfes befinden sich innen gegenüberliegend jeweils eine Art Rampe (siehe auch Abb. 15C), durch die die beiden Führungszangen (siehe auch Abb. 15B) leicht zusammengedrückt werden und nach einer leichten Drehung nach rechts in ihre Führung einschnappen.

Die Führung kann ebenfalls durch eine Kante realisiert werden, welche das Herausziehen des Druckknopfes verhindert. Die Führung begrenzt bevorzugt die Drehung des Knopfes auf 90 Grad.

Bei einem Winkel von 45 Grad befindet sich bevorzugt eine leichte Verjüngung des Radius (siehe auch Abb. 15D), so dass sich die Führungszangen im Betrieb nicht verdrehen können. Dies ist auch für den Benutzer fühlbar, so dass eine taktile Rückführung der Position entsteht.

Zur Demontage der Bauteile Druckknopf und Aufsatzhülse ist bevorzugt eine weitere Rampe am Anschlag für die Drehung nach links eingebaut. Diese beginnt bevorzugt in halber Höhe des Anschlags, so dass der Druckknopf halb eingedrückt sein muss. Hierdurch kann man die Führungszangen bevorzugt mittels der Rampe öffnen und diese können beim Herausziehen des Druckknopfes aus der Hülse über einen Anschlag hinübergleiten, wodurch es möglich ist, die Apparatur wieder zu demontieren.

Am unteren Teil der Aufsatzhülse direkt über der Fixierung für den Kolben können bevorzugt innen vier Leitstrukturen angebracht werden (sieh auch Abb. 15E). Diese dienen bevorzugt zur Führung einer Feder (siehe auch Abb. 16), die über die Steuerzangen Druck auf den Knopf ausübt. Der dadurch entstandene Hubbereich des Knopfes gegen die Feder ist konstruktionsbedingt vorgebbar und kann den Erfordernissen angepasst werden.

Eine bevorzugte Montage der Dosiereinheit kann beispielsweise in folgenden Schritten erfolgen:
- Die Feder wird in die Aufsatzhülse eingeführt und nach unten geschoben
- Der Druckknopf wird so in die Aufsatzhülse eingeführt, dass die Führungszangen sich in der Position der Rampen auf der Innenseite der Aufsatzhülse befinden.
- Der Druckknopf wird vollständig eingeführt und nach rechts gedreht
- Nach 45 Grad ist ein leichter Widerstand spürbar
- Nach 90 Grad ist die Endposition erreicht

Damit ist die Dosiereinheit fertig montiert. Eine bevorzugte Demontage der Dosiereinheit kann beispielsweise in folgenden Schritten erfolgen:
- Der Druckknopf wird nach links um 90 Grad gedreht
- Bei 45 Grad ist ein leichter Widerstand spürbar
- Dann wird der Druckknopf eingedrückt und gleichzeitig ein wenig nach links gedreht
- Anschließend kann der Druckknopf herausgezogen werden

Zur Fertigstellung der Dosiereinheit bzw. des Kolbenaufsatzes mit Aufsatzhülse und Druckknopf wird bevorzugt zunächst die Kolbenstange in den Spritzenbehälter bis zur gewünschten Tiefe eingeschoben. Danach wird der Druckknopf des Dosieraufsatzes bzw. Kolbenaufsatzes nach links gedreht, die Kolbenstange in das untere Loch eingeführt und der Dosieraufsatz in den Spritzenbehälter gesteckt. Anschließen wird der Druckknopf um 90 Grad nach rechts gedreht. Damit ist die Dosiereinrichtung fertig montiert und das Substrat kann von vorn durch den Spritzenauslauf bzw. die Düse eingespritzt werden.

Die Funktion des bevorzugten Dosieraufsatzes bzw. Kolbenaufsatz basiert bevorzugt darauf, dass sich zwischen den Steuerzangen eine Kolbenstange mit Zacken (bzw. einer Zahnung) befindet und mit jedem Knopfdruck die Steuerzangen die Kolbenstange um eine feste Distanz herausschiebt. Der Rückweg der Führungszangen erfolgt durch die beim Druck gespeicherte Energie der Rückstellfeder. Das Mitgleiten des Kolbens bei dem Rückweg der Steuerzangen zur nächsten Zacke wird bevorzugt dadurch verhindert, dass der Kolben durch eine enge Passung im Spritzenkolben durch Reibung blockiert wird. Diese Reibungskräfte können zwar durch den Druck auf den Druckknopf und die Einleitung der Kraft mittels der Führungszangen überwunden werden, nicht aber durch die geringen Federkräfte und die formgünstige Gestaltung der Kolbenstange in der Bewegungsrichtung der Federwirkung.

Die bevorzugte Ausführungsform erlaubt mithin eine besonders zuverlässige und einfache Dosierung einer vorbestimmten Menge an Polymermischung, welche bevorzugt durch die Beabstandung einer Zahnung (bzw. der Zacken) auf der Kolbenstange des Spritzkolbens vorgegeben wird.

Bevorzugt kann die Feder nur bei der ersten Montage der Dosiereinheit eingeführt werden. Danach kann die Aufsatzhülse mit dem Druckknopf fest zusammen bleiben. Durch die 90 Grad Drehung kann man bewirken, dass die Aufsatzhülse von dem Spritzenkörper abziehbar ist und mithin auf einen neu befüllten Spritzenkörper aufsetzbar ist. Durch erneute Drehung um 90 Grad kann der Zangenmechanismus wieder greifen und die Vorrichtung ist für eine erneute dosierte Abgabe von Portionen bereit.

In einem weiteren Aspekt betrifft die Vorrichtung zudem einen Kit umfassend
- eine beschriebene Vorrichtung zur Applikation einer Polymerelektrode
- eine beschriebene lichthärtende Polymermischung
- optional Instruktionen zur Applikation der lichthärtenden Polymermischung mittels der Vorrichtung als Polymerelektrode auf der Haut.

Der Fachmann erkennt, dass bevorzugte Ausführungsformen und Vorteile, welche im Zusammenhang mit der lichthärtende Polymermischung oder der Vorrichtung zur Applikation einer Polymerelektrode offenbart wurden sich gleichermaßen auf den beanspruchten Kit übertragen.

In einer bevorzugten Ausführungsform kann der Kit daher beispielsweise zudem eine oder mehrere leitfähige Faser, bevorzugt Kunststofffasern, bzw. ein Textilkabel umfassen.

In einer bevorzugten Ausführungsform liegt die Polymermischung innerhalb des Spritzenbehälters vor. Beispielsweise können zwischen 1 ml und 100 ml, bevorzugt zwischen 1 ml und 20 ml der beschriebenen Polymermischung im Spritzenkörper vorliegen und unmittelbar für die Applikation mit als Polymerelektrode bereitgestellt werden.

### DETAILLIERTE BESCHREIBUNG

### FIGUREN

Im Folgenden soll die Erfindung an Hand von Figuren näher erläutert werden, ohne auf diese beschränkt zu sein.

### Beschreibung der Abbildungen

- Abb. 1: Abbildung dreier EEG-Elektroden auf menschlicher Haut.
- Abb. 2: Schlaf-EEG von freibeweglichen Ferkeln mit einer Hydrogel-Elektrode.
- Abb. 3: Schlaf-EEG von freibeweglichen Ferkeln mit einer PEDOT Polymer Elektrode
- Abb. 4: Messung von Impedanz und Phasenwinkel einer PEDOT Polymer Elektrode und einer Ag/AgCl Elektrode auf einer Agar Platte
- Abb. 5: Impedanz einer PEDOT Polymer Elektrode auf einer Haut.
- Abb. 6: Messung von Impedanz und Phasenwinkel einer PEDOT Polymer Elektrode vor und nach der Aushärtung mit Blaulicht.
- Abb. 7: Mechanische Bruchtests einer PEDOT Polymer Elektrode.
- Abb. 8: Spannungs-Dehnungsmessung von einer PEDOT Polymer Elektrode.
- Abb. 9: Kraftmessung von einer PEDOT Polymer Elektrode.
- Abb. 10: Vergleichsmessung von Impedanz und Phasenwinkel für eine Polymer Elektrode auf Basis eines PMMA-Zahnzements
- Abb. 11: Illustration der Anbringung einer Polymerelektrode bei Frühgeborenem im Vergleich zu Elektroden des Standes der Technik
- Abb. 12: Schematische Illustration eines Spritzkolbens mit einer Kolbenstange, welche eine Zahnung aufweist (links: 3D-Ansicht, rechts: Schnittansicht)
- Abb. 13: Schematische Illustration eines Druckknopfes mit einem Führungszangenpaar und einem Steuerzangenpaar (links: 3D-Ansicht, rechts: Längsschnitt, oben Querschnitt)
- Abb. 14: Schematische Illustration einer Rückstellfeder (links: 3D-Ansicht, rechts: Schnittdarstellung)
- Abb. 15: Schematische Illustration einer Aufsatzhülse mit Rückstellfeder (links: 3D- und Explosionszeichnung, rechts: Schnittdarstellung)
- Abb. 16: Illustration einer vollständig assemblierte Dosierspritze.
- Abb. 17: Illustration eines Bestrahlungsaufsatz zur Lichtaushärtung der flüssigen Polymermischung
- Abb. 18: Schematische Illustration einer bevorzugten Vorrichtung zur Applikation einer Polymerelektrode auf einer Haut

### Detaillierte Beschreibung der Abbildungen

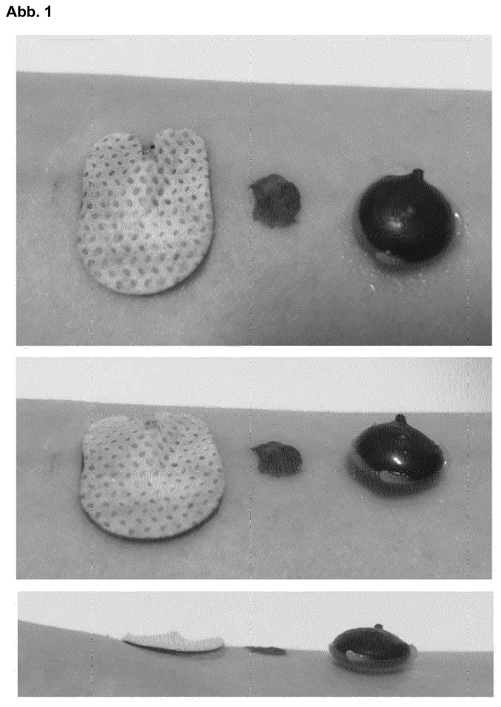

Abbildung 1 zeigt drei Typen von EEG Elektroden auf menschlicher Haut. Eine relativ flache Hydrogel Elektrode links, die PEDOT Polymer Elektrode mit 4 mm Durchmesser in der Mitte und rechts eine Ag/Ag/Cl Cup Elektrode mit leitfähigem Elektrodengel.

Abbildung 2 zeigt Schlaf EEGs von frei beweglichen, nicht fixierten Ferkeln. Eine K-Komplexartige Struktur ist sichtbar, aufgezeichnet mit einer Hydrogel Elektrode auf der Basis von Ag/AgCl in einer parietalen Position auf Höhe des somatosensorischen Cortex.

Abbildung 3 zeigt Schlaf EEGs von frei beweglichen, nicht fixierten Ferkeln. Eine K-Komplex artige Struktur ist sichtbar, aufgezeichnet mit einer selbst-haftenden PEDOT Polymer Elektrode in einer parietalen Position auf Höhe des somatosensorischen Cortex.

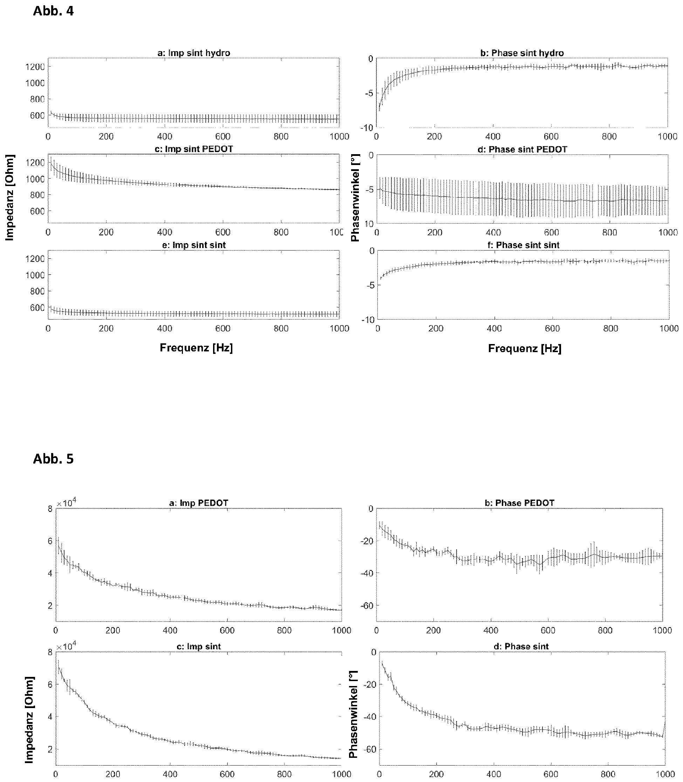

Abbildung 4 zeigt Impedanz und Phasenwinkel der PEDOT Polymer Elektrode und einer Ag/AgCl Elektrode auf einer Agar Platte mit physiologischer Kochsalz Konzentration. Zur Kontrolle werden zwei Ag/AgCl (sint) Elektroden mit 10cm Abstand voneinander gegeneinander gemessen (a,b, jeweils 10cm Abstand zwischen den Elektroden auf der Platte). Zudem wurde eine selbsthaftende Hydrogelelektrode gegen die Ag/AgCl Elektrode vermessen (sint,hydrogel, c und d). Die PEDOT Polymer Elektrode hat eine Oberfläche von 2x3mm, in etwa die Hälfte der Oberfläche der Ag/AgCl Elektrode. Die Impedanz der PEDOT Polymer Elektrode ist etwas höher als die der Vergleichselektroden (a und e). Der absolute drift des Phasenwinkels ist vergleichbar zwischen den Elektroden, allerdings zeigen die PEDOT Polymer Elektroden eine höhere Standardabweichung (d, Fehlerbalken) als die Vergleichselektroden (f,b).

Abbildung 5: Die Impedanz wurde von 10 bis 1000 Hz auf der Haut gemessen gegen eine zweite PEDOT Polymer Elektrode in 10cm Entfernung. Die gleiche Messung wurde mit zwei Ag/AgCl Elektroden durchgeführt. Beide Elektroden zeigen ähnliche Resultate.

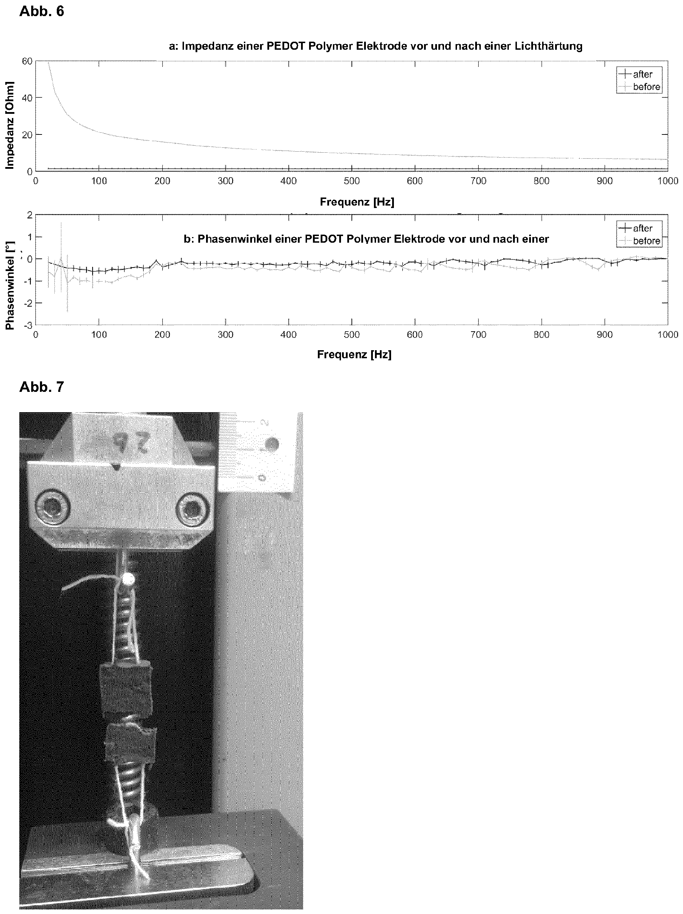

Abbildung 6 zeigt Impedanz und Phasenwinkel der PEDOT Polymer Elektrode vor und nach der Aushärtung mit Blaulicht. Nach der Belichtung mit Blaulicht reduziert sich die Impedanz der PEDOT Polymer Elektrode von etwa 60kOhm auf 1kOhm (obere Zeile, dunkle Linie nach der Belichtung). Auch eine reduzierte Varianz des Phasenwinkels kann nach der Belichtung beobachtet werden (untere Zeile, dunkle Linie nach der Belichtung). Fehlerbalken zeigen die Standardabweichung.

Abbildung 7 zeigt eine Fotographie der Bruchsituation während der mechanischen Testung der PEDOT Polymer Elektrode. Die Schnüre wurden in die Probe eingebettet und dienten als Verbindung zum Extensometer.

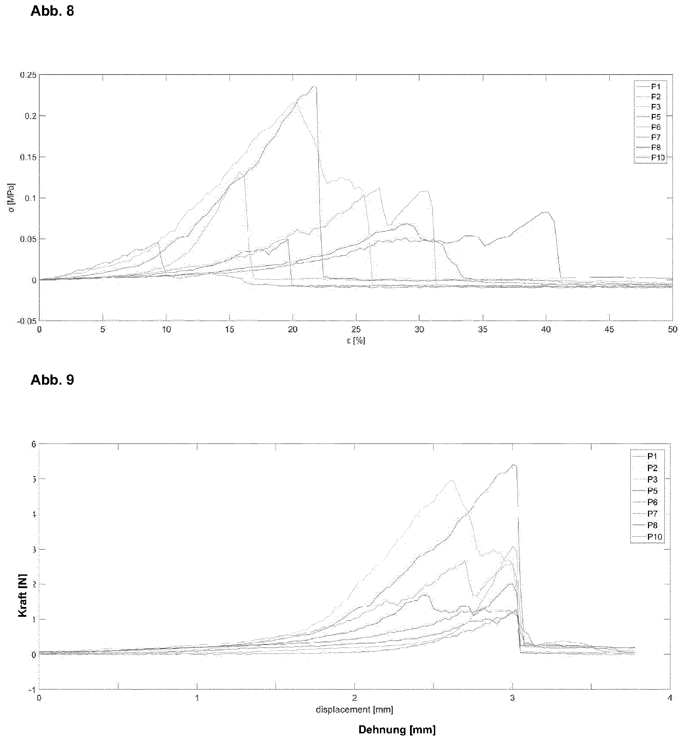

Abbildung 8 zeigt eine Spannungs-Dehnungsmessung für die PEDOT Polymer Elektrode. 10 gleich geformte Stücke der PEDOT Polymer Elektrode (10x2x20mm) wurden bis zur Zerstörung (starker Abfall der Kraft Sigma) gemessen. Auf der x-Achse ist die normalisierte Ausdehnung des Materials aufgetragen (epsilon), auf der Y-Achse die Kraft pro Fläche (sigma [MPa]).

Abbildung 9 zeigt eine Kraftmessung für die PEDOT Polymer Elektrode.10 gleich geformte Stücke der PEDOT Polymer Elektrode (10x2x20mm) wurden gemessen bis zur Zerstörung. Zum Vergleich des Verhältnisses zwischen Kraft und Dehnung sind die verschiedenen Messkurven am Zerstörungspunkt aligniert. Ein hoher Grad an Variabilität wird sichtbar, der im Aushärtungsverfahren mit einem dentalen Blaulichtgerät begründet sein kann. Zwei Proben wurden ausgeschlossen, wegen nicht kompletter Aushärtung (Nr. 4 und Nr.9).

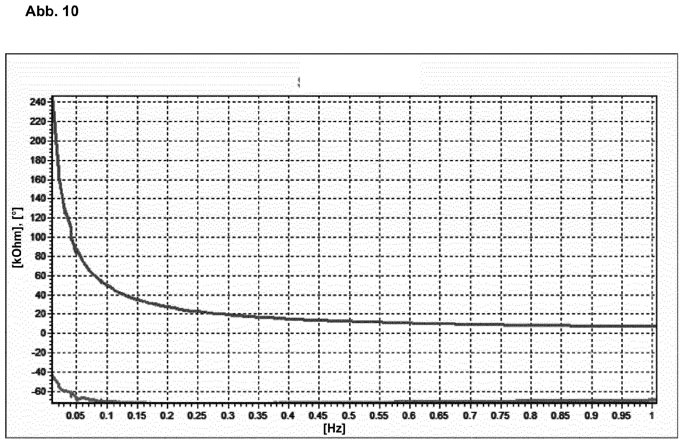

Abbildung 10 zeigt eine Impedanz und Phasenwinkelmessung für eine Polymer Elektrode auf Basis eines Methylmethacrylat basierenden Zahnzements. Die Vergleichspolymerelektrode basiert auf einer 1:1 Mischung von PMMA (Paladur Zahnzement) und PEDOT: PSS (Clevios SV3, Heraeus), welche auf Haut die appliziert und lichtgehärtet wurde. Zwei Elektroden mit einem Durchmesser von 1cm und 2mm Dicke werden im Abstand von 10cm auf dem Handrücken positioniert. In die Elektrode wird ein Metallkabel eingelassen und mit den 4 Pol Klemmen des Impedanzmessgerätes verbunden ist (LCR-6100, Gwinstek). Die Messung läuft von 10Hz bis 100 Hz. Die Vergleichspolymerelektrode verbleibt in einem Bereich von 7kOhm und höher, was deutlich höher ist als für die auf Dimethacrylaten basierende PEDOT Polymer Elektrode. Insbesondere im niedrigen Impedanzbereich wird sogar ein sehr hoher Widerstand von 100 kOhm erreicht.

Abbildung 11 illustriert die Verwendung der erfindungsgemäßen Polymerelektrode 3 für eine schonende Langzeit-EEG Ableitung. Bekannt EEG Cup-Elektroden 2. Diese Elektroden 2 sind relativ sperrig, und können Druckstellen und Schmerzen zu verursachen. Insbesondere könne die Cup-Elektroden nur dann mit einer hinreichenden Stabilität platziert werden, wenn sie in einer Kopfkappe bzw. Elektrodenhaube integriert sind, welche daher für Neu- oder Frühgeborenen als belastend sind. Auch ist eine Rasur der Haare bzw. des Flaums notwendig, um die Elektroden aufzubringen, was zusätzlichen Stress bedeutet.

Im Gegensatz dazu lässt sich die erfindungsgemäße Polymerelektrode 3 auf einfache Weise, selbst unter Haare auf der Haut des Frühgeborenen applizieren und führt zu ausgezeichneten Aufzeichnungsergebnisse auch bei einer EEG-Langzeitableitung. Eine Entfernung ist vorteilhafter schmerzfrei möglich.

Abbildung12 zeigt einen bevorzugte Ausführungsform eines Spritzenkolben 4, welcher eine Kolbenstange 5 mit einer Zahnung umfasst, wobei die Zahnung in Bezug auf den Umfang der Kolbenstange 5 bevorzugt in zwei gegenüberliegenden Bereichen vorliegt, welche von einem Bereich ohne Zahnung getrennt werden. Bevorzugt können die Bereiche mit bzw. ohne Zahnung jeweils abwechselnd ein Viertel (90°) des Umfanges einnehmen.

Die Zahnung ermöglicht es einem im Druckkopf integrierten Zangenpaar, den Spritzenkolben 4 entweder zu bewegen, oder aber über die Zacken (der Zahnung) hinwegzugleiten. Die Verhaltensweise hängt von der Winkelstellung der Kolbenstange 5 oder des Zangenpaares ab und ändert sich jeweils mit jeder 90 Grad Drehung. Der Abstand der Zacken (der Zahnung) bestimmt dabei den Vorschub und über das Spritzenvolumen die abgegebene Dosiermenge. Am Ende der Kolbenstange 5 befindet sich bevorzugt eine Spitze, die ein sicheres Einfädeln in das Zangenpaar ermöglicht.

Abbildung 13 zeigt eine bevorzugte Ausführungsform eines Druckknopfes 6. Der Druckknopf 6 besteht zum einen aus einem Knopf zum Drücken, zum anderen aus zwei Zangenpaaren 7 und 8. Das eine Zangenpaar (Steuerzangen 8) steuert bevorzugt die Kolbenstange, das andere Zangenpaar (Führungszangen 7) dient bevorzugt zur Fixierung des Druckknopfes 6 in der Aufsatzhülse. Dazu besitzen die Zangen 7 und 8 bevorzugt Haken, welche gegen die Innenfläche der Aufsatzhülse gerichtet sind. Eine Drehung der Zangenpaare kann erfolgen, indem man den Knopf dreht.

Abbildung 14 zeigt eine Rückstellfeder 9, welche wie beschrieben, bei Betätigung des Druckknopfes während der Dosierung Energie speichert, sodass ein Rückweg der Führungszangen durch die Rückstellfeder 9 erfolgt.

Die Abbildung 15 zeigt eine bevorzugte Ausführungsform einer Aufsatzhülse 10, welche am unteren Abschnitt zwei Passungen 11 für eine Fixierung der Aufsatzhülse 10 im Spritzenkörper aufweist. Im oberen Teil befindet sich eine Passung 12 für eine leichtgängige Führung des Druckknopfes. Als Anschlag dient bevorzugt eine Kante, die bei der Verjüngung des Innendurchmessers entsteht. Die Steuerung und Fixierung des Druckknopfes erfolgt durch Konturen in der Innenfläche der Aufsatzhülse 10. Zur Erleichterung der Montage des Druckknopfes befinden sich innen gegenüberliegend jeweils eine Rampe 13, durch welche die beiden Führungszangen leicht zusammengedrückt werden und nach einer leichten Drehung nach rechts in ihre Führung einschnappen.

Die Führung kann ebenfalls durch eine Kante realisiert werden, welche das Herausziehen des Druckknopfes verhindert. Die Führung begrenzt bevorzugt die Drehung des Knopfes auf 90 Grad. Bei einem Winkel von 45 Grad befindet sich bevorzugt eine leichte Verjüngung des Radius, so dass sich die Führungszangen im Betrieb nicht verdrehen können. Dies ist auch für den Benutzer fühlbar, so dass eine taktile Rückführung der Position entsteht.

Zur Demontage der Bauteile Druckknopf und Aufsatzhülse ist bevorzugt eine weitere Rampe am Anschlag für die Drehung nach links eingebaut. Diese beginnt bevorzugt in halber Höhe des Anschlags, so dass der Druckknopf halb eingedrückt sein muss. Hierdurch kann man die Führungszangen bevorzugt mittels der Rampe öffnen und diese können beim Herausziehen des Druckknopfes aus der Hülse 10 über einen Anschlag hinübergleiten, wodurch es möglich ist, die Apparatur wieder zu demontieren. Am unteren Teil der Aufsatzhülse direkt über der Fixierung für den Kolben können bevorzugt innen vier Leitstrukturen 15 angebracht werden, welche zur Führung der Rückstellfeder 9 dienen, die über die Steuerzangen Druck auf den Druckknopf ausübt. Der dadurch entstandene Hubbereich des Druckknopfes gegen die Rückstellfeder 9 ist konstruktionsbedingt vorgebbar.

Abb. 16 zeigt eine vollständig assemblierte Dosierspritze, umfassend eine Aufsatzhülse 10, einen Druckknopf 6 und einen Spritzkolben 4, welche in den Spritzenbehälter 16 eines Spritzenkörpers 18 mit Spritzenauslauf 17 eingebracht vorliegt.

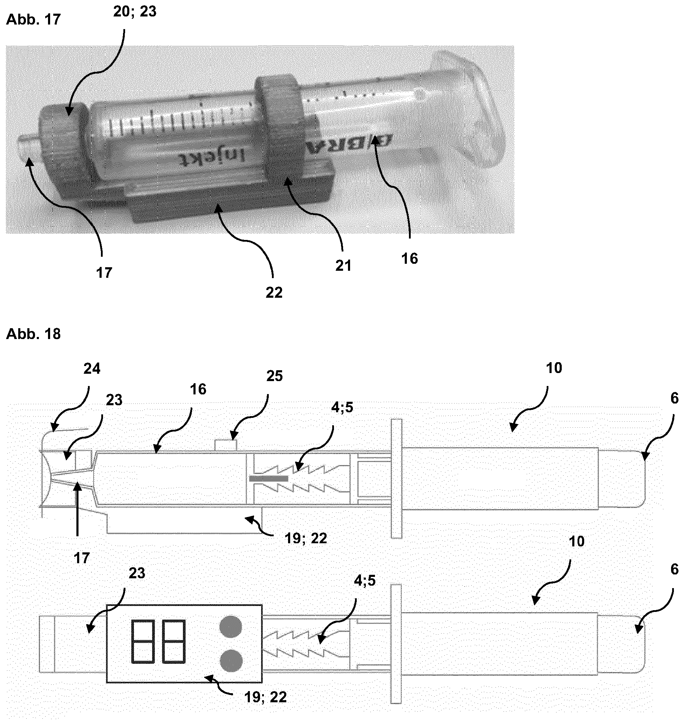

Abb. 17 illustriert einen Bestrahlungsaufsatz 19 zur Aushärtung der flüssigen Polymermischung mit blauem LED-Licht. Die LEDs sind dabei in dem vorderen Haltering 20 untergebracht, welche sich um den Spritzenauslauf 17 schließt Eine hinterer Haltering 21 dient der Arretierung am Spritzenbehälter 16. Eine elektronische Steuereinheit 22 kann in einen Steg zwischen den Halteringen 20, 21 eingebracht vorliegen.

Abb. 18 zeigt eine bevorzugte Ausführungsform einer Vorrichtung zur Applikation einer Polymerelektrode. Die Vorrichtung umfasst zu diesem Zweck einen Spritzenkörper mit einem Spritzenauslauf 17 und einem Spritzenbehälter 16 in welchem die leifähige Polymermischung eingebracht werden kann.

Die Vorrichtung umfasst weiterhin einen Kolbenaufsatz mit einem Spritzenkolben 4, welcher dafür eingerichtet ist, um im Spritzenbehälter 16 aufgenommen zu werden und eine Polymermischung aus dem Behälter 16 und durch den Spritzenauslauf 17 zu extrudieren. Vorzugsweise umfasst der Spritzenkolben 4 zu diesem Zweck einen Stopfen.

Durch Bewegen des Kolbens 4, an dem der Stopfen befestigt ist, wird die im ersten Volumen 16 befindliche Polymermischung in den nachgeschalteten Spritzenauslauf 17 geleitet. Die Vorrichtung ist dadurch gekennzeichnet, dass um den Spritzenauslauf 17 ein oder mehrere Lichtquellen, insbesondere LEDs 23 angeordnet sind. Die Anordnung der Lichtquellen 23 orientiert sich bevorzugt an der Geometrie des Spritzenauslaufes 17, wobei die Lichtquellen 23 für den dargestellten konischen Spritzenauslauf 17 bevorzugt ringförmig um selbigen angeordnet sind. Die automatisierte Aktivierung der Lichtquellen 23 anhand der Bewegung des Spritzenkolbens 4 führt zu einer optimierten zeitlichen Synchronisation des Ausstoßes und der Lichthärtung und somit zuverlässig zu hochqualitativen Polymerelektroden.

Die Lichtquellen werden von einer Steuereinheit 22 auf Basis eines Auslösesignal automatisch aktiviert und für eine bevorzugt vorbestimmte Beleuchtungszeit gesteuert. Zu diesem Zweck weist die gezeigte Vorrichtung einen Sensor 25 zur Detektion einer Bewegung des Spritzkolbens auf, wobei das Auslösesignal zur Aktivierung der Blaulichtquellen einer detektierten Bewegung des Spritzkolbens entspricht. Verschiedenen Sensoren können zu diesem Zweck eingesetzt werden. Beispielsweise können induktive oder kapazitative Näherungssensoren verwandt werden. Diese können beispielsweise den Abstand zwischen einer Position auf dem Spritzkolben 4 und einer Position auf dem Spritzbehälter 16 bestimmen und somit eine relative Bewegung erfassen. In bevorzugt umfasst der Sensor 25 einen Neodynmagnet vor, dessen Magnetfeld von einem Detektor erfasst werden kann.

Die bevorzugt gezeigt Vorrichtung umfasst zudem Positioniermittel zur Einbringung einer oder mehrere leitfähiger Fasern 24, bevorzugt einer oder mehrerer leitfähiger Kunststofffasern in die Polymerelektrode. Bei den Positioniermitteln zur Einbringung der leitfähigen Fasern kann es sich bevorzugt um eine Öse handeln, durch welche die leitfähigen Fasern bzw. das Textilkabel 23 in Bezug auf den Spritzenauslauf 17 positioniert werden. Dies erlaubt eine besonders einfache Handhabung der Vorrichtung zur Bereitstellung einer Polymerelektrode mit bereits integriertem Ableitungskabel.

### BEISPIELE

Im Folgenden soll die Erfindung anhand von Beispielen näher erläutert werden. Die Beispiele dienen der Illustration bevorzugter Ausführungsform der Erfindung ohne diese zu beschränken.

Die Experimente und Beispiele zeigen, dass die beanspruchte Polymerelektrode sich durch eine zuverlässige Aufbringung, hohen Tragekomfort und Aufzeichnungsqualitiät auszeichnet. Hierdurch werden auch schonende Langzeitableitungen, beispielsweise für Langzeit-EEGs als Routineuntersuchung für frühgeborene Kinder, ermöglicht, wobei insbesondere eine schmerzfreie Applikation unter Haaren bzw. Flaum, wie beispielsweise bei Frühgeborenen oder Tieren, gewährleistet werden kann.

### Material und Methoden für die Beispiele

### Tierexperimente:

Es wurden EEG Messungen von 6 neugeborenen Ferkeln genommen. Alle Vorgänge wurden durch die lokale Ethikkommission(#23177-07/G10-1-010/G 15-15-011, Landesuntersuchungsamt Rheinland-Pfalz, Germany) genehmigt und sind imEinklang mit den europäischen sowie nationalen Regularien (European Communities Council Directive, 86/609/ECC;
Tierschutzgesetz). Alle Tierbehandlungen erfolgten im Einklang mit den [Medical Center of the Johannes Gutenberg University Mainz] Tierschutzregularien. Die Ferkel wurden beruhigt, indem sie in ein Handtuch gewickelt wurden, ohne Sedierung. Die Elektrodenapplikation dauerte etwa 5-10 Minuten. Die PEDOT Polymer Elektrode wurde fixiert, wie unten beschrieben. Danach wurde das Kabel der Elektrode mit einem 1-Kanal EEG Telemetriesystem verbunden mit einer Größe von ca. 1x2cm und einem Gewicht von 10g. Das System wurde mit einem ungiftigen Körpersilikon fixiert (Body Double fast, Smooth-On Inc., USA) direkt auf dem Kopf, oberhalb der Elektroden.

### EEG Ableitungen

Wegwerfbare, selbsthaftende Hydrogelelektroden mit Ag/AgCl (Spes Medica S.r.l., Genova, Italy) wurden über dem Cerebellum (zwischen den Ohren) zur Erdung, über der Nase als Referenz und zwischen Auge und Ohr positioniert, um vom rechten somatosensorischen Cortex abzuleiten (parietale Position). Vor der Fixierung der Elektrode wurde die Haut im Fall der Hydrogelelektroden rasiert und die Haut wurde mit einem abrasiven Gel gereinigt (Abralyt HiCl, Easycap GmbH, Herrsching, Germany) um tote Hautzellen zu entfernen und dadurch die Impedanz zu verringern.

Die Polymer Elektrode basiert auf einer 1:1 Mischung von PEDOT:PSS (Heraeus, Clevios SV3) und dem Zahnzement Tetric Evo Flow^{®} (ivoclar vivadent). Die erhaltene Polymermischung kann, wie beschrieben, mittels Lichthärtung der Bereitstellung einer Polymerelektrode dienen. Eine derart bevorzugte Polymerelektrode wird in den Abbildungen und Beispielen auch als PEDOT Polymer Elektrode bezeichnet und kennzeichnet die mithin besonders bevorzugte Polymerelektrode auf Basis von PEDOT:PSS und einen auf Dimethacrylat basierenden Zahnzement (hier Tetric Evo Flow^{®}). Vergleichbare Ergebnisse werden auch unter Verwendung von Ionoseal^{®} als ein auf Dimethacrylat basierenden Zahnzement erzielt, wobei die Langzeitstabilität der Polymerelektroden sogar noch gesteigert werden kann.

Die lichthärtende PEDOT Polymer Elektrode wurde auf frei laufenden Ferkeln im Stall appliziert. Ein Silberdraht wurde an der Spitze entisoliert und an der gewünschten Position fixiert. Die PEDOT Polymer Elektrode wurde im gelförmigen Zustand auf die entisolierte Seite des Kabels aufgetragen und mit Blaulicht für 20s ausgehärtet. Für die Lichthärtung wurde eine dentale Blaulichtlampe genutzt (Drs Light Clever DUAL, 400-490 nm, 1500 mW/cm2). Die Distanz zum Gel (d.h. die Polymermischung aus PEDOT und dem Zahnzement auf Dimethacrylatbasis) sollte bevorzugt ca. 0,5 bis 1cm betragen. Das Gel kann unter Haaren aufgetragen werden, daher war eine Rasur nur im Fall der Hydrogel Elektroden bei der Kontrollgruppe notwendig. Die Daten wurden aufgenommen und gesendet durch ein 1 Kanal Telemetrie System (mit einem AC gekoppelten Verstärker und einer sampling rate von 500 Hz, Quelle 21). Nur EEG Phasen ohne Artefakte wurden zur weiteren Analyse herangezogen. Die Dicke der PEDOT Polymer Elektrode beträgt etwa 0,5 mm, der Durchmesser 5mm mit runder Fläche.

### EEG Analyse:

Die Daten wurden mit Matlab (2016b, Simulink) und mit brainstorm analysiert. Die EEG Rohdaten wurden mit einem digitalen Butterworth Filter dritter Ordnung in die Frequenzbänder zerlegt. Die normierte Grenzfrequenz wurde für Bänder delta [0-4 Hz], theta [4-8 Hz], alpha [8-13 Hz], beta [13-30 Hz], low-gamma [30-80 Hz] and high-gamma [80-120 Hz] berechnet. Wn ist eine Zahl zwischen 0 und 1, wobei 1 die Nyquist Frequenz darstellt (halbe Aufnahmerate/sampling rate, hier 250Hz für die heruntergesampelten EEG Daten). Die Zähler und Nenner Werte (IIR Filter) wurden mit der Funktion butter errechnet und mit der matlab Funktion filtfilt verwendet um die EEG Daten zu filtern. Für das EEG Band delta [0-4Hz] wurde ein Tiefpassfilter eingesetzt. Alle anderen EEG Bänder wurden mit einem Bandpass Filter design extrahiert. Um relative Unterschiede der Repräsentation der Stärke verschiedener Bänder zu erkennen, wurde die normierte Differenz der Bandstärke berechnet.

### Statistik:

Die Verteilung der Daten wurde mit dem Lilliefors test (Matlab 2016b, Simulink) getestet. Tests mit mehr als zwei Gruppen wurden mit dem nicht-parametrischen Kruskal Wallis Test oder einer Anova (bei Normalverteilung der Daten) sowie jeweils einem angeschlossenen multiple comparison durchgeführt um die exakten statistischen Beziehungen zwischen den Gruppen zu betrachten. Alle Tests sind in der matlab Statistik toolbox implementiert (2016b, Simulink). Bei dem Vergleich von nur zwei Gruppen wurde der non parametrische Wilcoxon Rangsummentest verwendet.

### Impedanzmessungen:

Die Impedanzmessungen wurden mit einem Red Pitaya (Impedance analyser module, Stem lab, Solkan, Slovenien) durchgeführt. Die PEDOT Polymer Elektrode wurde auf der Haut platziert und gegen eine zweite PEDOT Polymer Elektrode mit 10 cm Entfernung gemessen. Die gleiche Messung wurde mit Ag/AgCl Elektroden in Kombination mit Gel (Ten-20, Weaver, USA) durchgeführt. Die Impedanzmessung wurde von 10 bis 1000 Hz durchgeführt auf einer logarithmischen Skala. Pro Elektrode wurden 5 Messungen durchgeführt. Die Messung wurde 30min nach dem Aufbringen der Elektrode auf der Haut durchgeführt.

Zudem wurde die Impedanz aller drei Elektroden (PEDOT Polymer, gesinterte Ag/AgCl und Hydrogel) jeweils gegen eine gesinterte Ag/AgCl Elektrode auf einer Agarplatte mit physiologischer Kochsalzkonzentration (3% Agar in 150mM Kochsalz Lösung) gemessen. Zwischen den Elektroden wurde ein Abstand von 10cm gewählt. Die gesinterten Ag/AgCl Elektroden haben Abmessungen von 5mm Durchmesser und 5mm Dicke (runde Form), die Hydrogelelektroden haben eine ovale Form mit 10x8mm und 2mm Dicke. Die PEDOT Polymer Elektroden haben eine runde Form mit einem Durchmesser von 3mm und 2mm Dicke.

Für die Messung der Impedanz vor und nach der Lichthärtung wurde ein LCR-6100 (GW Instek, Taiwan) verwendet. Die PEDOT Polymer Elektrode wurde in der Mitte positioniert mit jeweils einer 5cm langen, leitenden Spur aus PEDOT:PSS, an welche die Kelvin Klemmen angeschlossen wurden um die 4 Pol Impedanzmessung von 10 bis 1000 Hz durchzuführen.

### DC drift

Um die Stabilität der PEDOT Polymer Elektrode unter DC (Gleichstrom) Bedingungen beurteilen zu können, wurde eine Testmessung bei einer Versuchsperson durchgeführt. An jeder Ableitstelle auf dem Kopf des Probanden wurden eng aneinander, aber ohne Kurzschluss eine PEDOT Polymer Elektrode und eine gesinterte Ag/AgCl Elektrode in Kombination mit Gel (1020) positioniert. Die Daten wurden mit einem medizinisch zugelassenen full band DC EEG System der Firma Neuroconn (Ilmenau, Deutschland) aufgenommen (Neuroprax). Die Aufnahmedauer betrug 10 Minuten. Es wurden nur solche Elektroden in die Analyse einbezogen, die von dem System als gut eingestuft wurden (basierend auf dem Signal zu Rausch Verhältnis). Um keine Einflüsse auf die laufende Messung zu haben, wird keine Messspannung angelegt. Der Drift von jeweils 6 Elektroden wurde berechnet, indem die Differenz aus Anfangs und Endwert berechnet wurde.

### Spannungsdehnungsmessung:

10 PEDOT Polymer Proben mit einer Größe von 10x2x20mm wurden mit Hilfe einer Polypropylen Form hergestellt. Vor der Belichtung wurde ein 10cm langer Polyethylen Faden (Nikol Weber KG, Selbitz, DE), in jede Probe eingelassen und dienten als Halterung im Extensometer. Für Abbildung 9 wurden die Messkurven am Punkt der maximalen Elongation der Polypropylen Faser zusammengeführt. An diesem Punkt wirkt die größte Kraft auf die Probe. Es wurde eine Testmessung durchgeführt, um die Elongation der Polypropylen Faser beurteilen zu können. Die Faser hat einen extrem steilen Kraftanstieg (6N für 0.4 mm Elongation) im Gegensatz zu den PEDOT Polymer Proben.

### Beispiel 1 - EEG Aufnahmen freilaufender Ferkel

Um relative Unterschiede in der Repräsentation der EEG Bänder durch die verschiedenen Elektrodentypen beurteilen zu können, wurde die normierte Differenz zwischen der EEG Band Power der PEDOT Polymer Elektrode und der Band Power der Ag/Ag/Cl Hydrogel Elektrode kalkuliert. Um etwaige Asymmetrien in der Repräsentation der spektralen Band Power durch die jeweilige Elektrode erkennen zu können, wurde die normierte Differenz zwischen den Bändern verglichen. Falls die verschiedenen EEG Bänder durch beide Elektroden gleichwertig dargestellt werden sollten, sollte die normierte Differenz zwischen den Bändern nicht unterschiedlich sein. In Tabelle 1 und 2 ist zu erkennen, dass es tatsächlich keine statistisch signifikanten Unterschiede der spektralen Power Differenz zwischen den EEG Bändern gibt.

Mit beiden Elektroden konnten wir typische EEG Muster wie spindle bursts, delta brushes oder k-Komplex artige Muster bei schlafenden oder ruhenden Ferkeln im Schweinestall ableiten (Abb. 2 und 3).

### Beispiel 2 - Impedanzmessungen

Die Impedanz wurde in einem Bereich von 10Hz bis 1000Hz gemessen und resultierte in Werten von 1.2 bis 0.8kOhm für die PEDOT Polymer Elektrode (Abb. 4c). Die Silber Elektrode hatte im Vergleich eine Impedanz von 0.5kOhm. Veränderungen der Phasenwinkel während der Messung sind mit allen Elektroden zu beobachten. Die Silber Hydrogel Elektrode (hydro) zeigt eine Veränderung von etwa 5° während der Messung (Abb. 4b). Die gesinterte Ag/AgCl Elektrode (sint) zeigte einen veränderten Phasenwinkel von 3° (Abb. 4f). Im Gegensatz dazu zeigte die PEDOT Polymerelektrode nur 2° Veränderung des Phasenwinkels, allerdings eine vergleichsweise hohe Standardabweichung (Fehlerbalken in Abb. 4d).

Des Weiteren wurde die Impedanz auf der Haut gemessen (Handrücken). Die PEDOT Polymer Elektrode zeigt hierbei, im Vergleich zu den Messungen auf der Agarose Oberfläche, verhältnismäßig niedrige Widerstände (Abb. 5a) und niedrigere Änderungen des Phasenwinkels (Abb. 5b) als die gesinterte Ag/AgCl Elektrode in Kombination mit Elektrodengel (Abb 5c,d). Die Standardabweichung ist hierbei für beide Elektroden vergleichbar.

Die Impedanz (Abb. 6a) und der Phasenwinkel (Abb. 6b) der PEDOT Polymer Elektrode wurden auch vor- und nach der Lichthärtung gemessen (n=4). Hierbei war die Impedanz relativ hoch vor der Lichthärtung [before curing: 10 Hz = 59 kOhm (Standardabweichung(SD) 0.2), 100 Hz = 20 kOhm (SD 0.2), 500 Hz = 11 kOhm (SD 0.2); nach der Belichtung: 10 Hz = 1.4 kOhm (SD 0.2), 100 Hz = 1.4 kOhm (SD 0.2), 500 Hz = 1.4 kOhm (SD 0.2)], und der Phasenwinkel zeigte eine höhere Varianz vor der Lichthärtung [vor der Belichtung: 10 Hz = -0.6° (SD 0.7), 100 Hz = -1° (SD 0.03), 500 Hz = -0.5° (SD 0.04); nach der Belichtung: 10 Hz = -0.2° (SD 0.05), 100 Hz = -0.6° (SD 0.1), 500 Hz = -0.2° (SD 0.08)] (Fig. 6).

### Beispiel 3 - Drift während der full band DC EEG Ableitung:

Weiterhin wurde die Stabilität der PEDOT Polymer Elektroden während einer full band DC EEG Ableitung untersucht. Dazu wurde eine EEG Aufnahme mit Standard Ag/AgCl Elektroden in Kombination mit einem geeigneten Elektrodengel (1020 Elektrodencreme) und direkt daneben mit PEDOT Polymer Elektroden durchgeführt. Es gab keine statistisch signifikanten Unterschiede im DC drift zwischen der PEDOT Polymer Elektrode und der Ag/AgCl Elektrode in Kombination mit einem Elektrodengel (alpha=0.01, p-value 0.4452, Wilcoxon Rank-Sum Test, Matlab 2016b).

### Beispiel 4 - Mechanische Tests:

Im Hinblick auf die gewünschte Haftung und gleichzeitig schmerzfrei Applikation und Entfernung wurden mechanische Tests für die gehärtete Polymerelektrode durchgeführt. Das Material ist weich und hat eine spröde Bruchcharakteristik. Der Bruch findet in der Mitte der Proben nach einer Elongation von 10 bis 40% (Abb.7) statt. Der Druck bis zum Bruch liegt zwischen 0.05 und 0.2 MPa (Abb. 8). Um eine bessere Vergleichbarkeit zu gewährleisten, wurden die Daten am Bruchpunkt zusammengelegt (Abbildung 9). Es ist eine hohe Varianz von Kraft und Dehnung erkennbar. Zwei Proben (Nr. 4 und 9) wurden aussortiert, da die unvollständige Aushärtung des Materials nach dem Bruch sichtbar wurde.

### Beispiel 5 - Vergleichsexperiment mit Polymerelektroden auf Basis eine Methylmethacrylat basierenden Zahnzements

Um die Aufzeichnungsqualität einer erfindungsgemäß bevorzugten Polymerelektrode umfassend PEDOT und ein auf Dimethacrylat basierendem Zahnzement mit einer Polymerelektroden auf Basis eine Methylmethacrylat basierenden Zahnzements zu testen, wurde ein Vergleichsexperiment durchgeführt. Die Vergleichspolymerelektrode wurde auf Basis einer 1:1 Mischung von einem auf Polymethylmethacrylat (PMMA) basierendem Zahnzement (Paladur Zahnzement) und PEDOT: PSS (Clevios SV3, Heraeus) gebildet. Die Applikation der Polymermischung auf der Haut und anschließende Lichthärtung erfolgt, wie beschrieben. Zwei Elektroden mit einem Durchmesser von 1cm und 2mm Dicke wurden im Abstand von 10cm auf dem Handrücken positioniert. In die Elektrode wurde ein Metallkabel eingelassen und mit den 4 Pol Klemmen des Impedanzmessgerätes verbunden (LCR-6100, Gwinstek). Die Messung lief von 10Hz bis 100 Hz.

Wie die Abb. 10 zeigt, bleibt die Impedanz (obere Spur) der Vergleichselektrode im Bereich von 7kOhm und höher, was deutlich höher ist, als die auf Dimethacrylaten basierende PEDOT Polymer Elektrode. Im niedrigen Impedanzbereich wird ein sehr hoher Widerstand erreicht um die 100 kOhm. Dies erschwert Biosignalmessungen. Zudem haben die Elektroden eine glatte, sehr harte Textur, die in sich stabil ist, aber schlecht auf der Haut haftet. Dies kann ein Grund für den höheren Widerstand sein im Gegensatz zur auf Dimethacrylaten basierenden PEDOT Polymer Elektrode.

Die Beispiele zeigen, dass gemäß der Lehre der Erfindung eine nicht-metallische EEG Elektrode bereitgestellt werden kann, die ihren Aggregatzustand durch die Applikation von Licht innerhalb weniger Sekunden ändert. Mit dieser Elektrode können wichtige Voraussetzungen für stabile Langzeit Ableitungen erfüllt werden.

Erstens, verliert die Polymerelektrode nicht ihre Funktionalität durch Austrocknung, wie es bei allen Standardelektroden der Fall ist, die in Kombination mit einem Elektrodengel genutzt werden. Außerdem ist die Polymerelektrode selbst haftend, was bisher bei keiner anderen trockenen Elektrode der Fall ist. Die Änderung des Aggregatzustandes ermöglicht die Verwendung sehr kleiner Mengen des ungehärteten Gels (bzw. der Polymermischung) unter den Haaren, ohne das eine Rasur notwendig wäre. Nach der Aushärtung durch Blaulicht, direkt auf der Haut, ist die Polymerelektrode hochgradig leitfähig und stabil aber nahezu unsichtbar. Dies macht die Elektrode besonders interessant für den täglichen Gebrauch, z.B. bei der Epilepsie Diagnostik oder der Schlaganfall Prävention.

Die miniaturisierte und flache Dimensionierung der Elektrode macht sie sehr komfortabel für den Träger (Abb.1). Ein wichtiger Punkt auch im Fall von Kleinkindern, in der neonatologischen Intensivstation oder für Schlaflabore. Die Elektrode kann durch seitlichen Druck einfach gelöst werden. Wir haben auch mechanische Tests der Elektrode durchgeführt (Abb. 7-9). Der relativ hohe Grad an Standardabweichung könnte auf die Härtungsmethode mit einer Dental Blaulicht LED zurückzuführen sein. Die Probengröße von 10x2x20mm wurde durch das experimentelle Design vorgegeben. Für die Anwendung beim EEG ist die Elektrodenfläche wesentlich kleiner (Abb.1). Die geringen Kräfte, die notwendig sind um das Material zu zerstören garantieren, auch eine schmerzfreie Ablösung von der Haut.

Trotzdem ist die Haftkraft groß genug für eine enge Hauthaftung. Reste der Elektrode können ausgebürstet werden. Auch andere Bioparameter können mit der Elektrode gemessen werden, wie Electrocardiographie, Electromyographie oder Electroenterographie. Die Ergebnisse der EEG Messungen bei frei laufenden Ferkeln im Schweinestall sind sehr ähnlich den Messungen mit Standard Ag/AgCl Elektroden (Abb. 2, 3). Die Funktionalität der PEDOT Polymer Elektrode wird bestimmt durch die enge Haftung auf der jeweiligen Oberfläche. Im Fall der Agarose Oberflächen bestand ein geringer Kontakt zwischen der Agarose und der PEDOT Polymer Elektrode, worin auch die relativ hohe Impedanz und die hohe Standardabweichung für die Phasenwinkel begründet sein könnten. Daher wurde die Impedanz auch auf menschlicher Haut gemessen (Abb. 5). In diesem Fall sind die Ergebnisse für die Impedanz und die Phasenwinkel sehr ähnlich für die PEDOT Polymer Elektrode und die gesinterte Ag/AgCl Elektrode. Zudem zeigt die PEDOT Polymer Elektrode sehr ähnliche DC drift Charakteristik wie die gesinterten Ag/AgCl Elektroden bei einer full band DC EEG Messung mit einer Testperson. Dies macht die Elektrode besonders interessant für Anwendungen auf der neonatologischen Intensivstation, da die langsamen Wellen des EEGs eine besondere Rolle bei der Hirnreifung spielen (vgl. Abb. 11).

**Tabelle 1: Mittlere spektrale Power für beide Typen von Elektroden, genutzt für EEG Aufnahmen im Schweinestall (PEDOT Polymer Elektrode und selbsthaftende Hydrogelelektrode) und Werte für die normierte Differenz sowie Standardabweichung.**

| **Frequenz Band** | **Mittelwert Power [µV²/ms]** | **Mittelwert normierte Differenz** | **Standardabweichung der normierten Differenz** |
|---|---|---|---|
| delta | Hydrogel: 1.18 | 0.15 | 0.09 |
| | PEDOT: 1.17 | | |
| theta | Hydrogel: 2.74×10⁻⁵ | 0.38 | 0.32 |
| | PEDOT: 1.33×10⁻⁴ | | |
| alpha | Hydrogel: 5.92×10⁻⁵ | 0.38 | 0.32 |
| | PEDOT: 2.83×10⁻⁴ | | |
| beta | Hydrogel: 7.76×10⁻⁵ | 0.39 | 0.32 |
| | PEDOT: 3.74×10⁻⁴ | | |
| gamma | Hydrogel: 1.14×10⁻⁴ | 0.41 | 0.32 |
| | PEDOT: 5.53×10⁻⁴ | | |
| high-gamma | Hydrogel: 3.00×10⁻⁵ | 0.45 | 0.35 |
| | PEDOT: 1.75×10⁻⁴ | | |

**Tabelle 2: Ergebnisse des multiple comparison Test (Matlab) basierend auf der stats Matrix des Anova Tests (Matlab 2016b), nach einem Lilliefors Test (Matlab 2016b) zur Abklärung der Verteilung (Normalverteilung liegt vor). Wenn das Konfidenzintervall (CI) nicht den Wert 0 umspannt, ist die Differenz statistisch signifikant. Es wurden keine statistisch signifikanten Unterschiede für die EEG Power Schwankungen zwischen den EEG Bändern für beide Elektroden.**

| **Gruppe1** | **Gruppe2** | **Unteresr CI** | **Dif Gruppen** | **Oberes CI** | **p-Wert** |
|---|---|---|---|---|---|
| delta | theta | -0.88 | -0.23 | 0.41 | 0.76 |
| delta | alpha | -0.87 | -0.23 | 0.41 | 0.77 |
| delta | beta | -0.89 | -0.24 | 0.40 | 0.73 |
| delta | gamma | -0.91 | -0.26 | 0.38 | 0.65 |
| delta | hgamma | -0.95 | -0.31 | 0.34 | 0.50 |
| theta | alpha | -0.64 | 0.00 | 0.65 | 1.00 |
| theta | beta | -0.65 | -0.01 | 0.63 | 1.00 |
| theta | gamma | -0.68 | -0.03 | 0.61 | 1.00 |
| theta | hgamma | -0.72 | -0.08 | 0.57 | 1.00 |
| alpha | beta | -0.66 | -0.01 | 0.63 | 1.00 |
| alpha | gamma | -0.68 | -0.03 | 0.61 | 1.00 |
| alpha | hgamma | -0.72 | -0.08 | 0.57 | 1.00 |
| beta | gamma | -0.66 | -0.02 | 0.62 | 1.00 |
| beta | hgamma | -0.71 | -0.06 | 0.58 | 1.00 |
| gamma | hgamma | -0.69 | -0.04 | 0.60 | 1.00 |

### LITERATUR

Alba, N. A., Sclabassi, R. J., Sun, M. & Cui, X. T. Novel hydrogel-based preparation-free EEG electrode. IEEE Trans. Neural Syst. Rehabil. Eng. 18, 415-423 (2010).
Attin, T., Vataschki, M., Hellwig, E.: Properties of resin-modified glassionomer restorative materials and two polyacid-modified resin composite materials. Quintessence Int 27: 203 (1996).
Balint, R., Cassidy, N. J. & Cartmell, S. H. Conductive polymers: Towards a smart biomaterial for tissue engineering. Acta Biomater. 10, 2341-2353 (2014).
Dr. Roland Frankenberger und Dr. Norbert Kämer, Glasionomerzemente, Wolfgang-M. Boer (Hrsg.): Metallfreie Restaurationen, Band 1, Teil 4, Spitta Verlag, Balingen 1999.
Ferree, et al." Scalp electrode impedance, infection risk, and EEG data quality" Clinical Neurophysiology 112 (2001): 536-544.
Guggenberger, R., May, R., Stefan, K.-P.: New trends in glass-ionomer chemistry. Biomaterials 19: 479 (1998).
Howson et al. "Born Too Soon: Preterm birth matters" Reproductive Health 10 (2013):
James, F. M. et al. Diagnostic Utility of Wireless Video-Electroencephalography in Unsedated Dogs. J. Vet. Intern. Med. 31, 1469-1476 (2017).
James, F. M. et al. Investigation of the use of three electroencephalographic electrodes for longterm electroencephalographic recording in awake and sedated dogs. Am. J. Vet. Res. 72, 384-90 (2011).
Kiilerich-Pedersen K: "Polymer Based Biosensors for Pathogen Diagnostics" In: "Environ-mental Biosensors", 1. Juli 2011.
Kim, D., Yeon, C. & Kim, K. Development and Experimental Validation of a Dry Non-Invasive Multi-Channel Mouse Scalp EEG Sensor through Visual Evoked Potential Recordings. Sensors. 17, 326 (2017).
Krachunov, S. & Casson, A. J. 3D Printed Dry EEG Electrodes. Sensors. 16, 1635 (2016).
Li, Q. et al. Highly conductive PEDOT:PSS Transparent Hole Transporting Layer with Solvent Treatment for High Performance Silicon/Organic Hybrid Solar Cells. Nanoscale Res. Lett. 12, 506 (2017).
Lloyd, R. O., Goulding, R. M., Filan, P. M. & Boylan, G. B. Overcoming the practical challenges of electroencephalography for very preterm infants in the neonatal intensive care unit. Acta Paediatr. 104, 152-157 (2015).
Löfhede, J., Seoane, F. & Thordstein, M. Textile Electrodes for EEG Recording - A Pilot Study. Sensors. 12, 16907-16919 (2012).
Lundt, A. et al. EEG Radiotelemetry in Small Laboratory Rodents: A Powerful State-of-the Art Approach in Neuropsychiatric, Neurodegenerative, and Epilepsy Research. Neural Plast., https://doi.org/10.1155/2016/8213878 (2016).
Manhart, J.: Charakterisierung direkter zahnärztlicher Füllungsmaterialien für den Seitenzahnbereich - Alternativen zum Amalgam? Die Quintessenz 2006; 57 (5): 465-481. (Zusammenfassung).)
Miriani, R. M., Abidian, M. R. & Kipke, D. R. Cytotoxic Analysis of the Conducting Polymer PEDOT using Myocytes. Conf. Proc. IEEE Eng. Med. Biol. Soc. 2008, 1841-1844 (2008).
Rozlosnik, N. New directions in medical biosensors employing poly(3,4-ethylenedioxy thiophene) derivative-based electrodes. Anal. Bioanal. Chem. 395, 637-645 (2009).
Tallgren,P., Vanhatalo,S., Kaila,K., and Voipio,J. (2005). Evaluation of commercially available electrodes and gels for recording of slow EEG potentials. Clinical Neurophysiology 116, 799-806.
Vanhatalo, S., Voipio, J. & Kaila, K. Full-band EEG (fbEEG): a new standard for clinical electroencephalography. Clin. EEG. Neurosci. 36, 311-317 (2005)
Zimmerli, B. et al. Schweiz Monatsschr Zahnmed 120: 980-986 (2010).

### BEZUGSZEICHEN

- 2: Bekannte EEG Cup-Elektroden
- 3: Polymerelektrode
- 4: Spritzkolben
- 5: Kolbenstange
- 6: Druckknopf
- 7: Führungszangen
- 8: Steuerzangen
- 9: Rückstellfeder
- 10: Aufsatzhülse
- 11: Passungen für Fixierung im Spritzenkörper
- 12: Passung für Führung für den Druckknopf
- 13: Rampe für den Druckknopf
- 14: Widerstand Drehbewegung
- 15: Führung für die Rückstellfeder
- 16: Spritzenbehälter
- 17: Spritzenauslauf
- 18: Spritzenkörper
- 19: Bestrahlungsaufsatz
- 20: erster Haltering
- 21: zweiter Haltering
- 22: Steuereinheit
- 23: Lichtquellen, insbesondere LEDs
- 24: leitfähige Fasern, insbesondere Textilkabel
- 25: Sensor zur Detektion der Bewegung des Spritzenkolbens, insbesondere umfassend einen Neodynmagnet

## Patentansprüche

1. Lichthärtende Polymermischung zur Herstellung von Polymerelektroden
**dadurch gekennzeichnet, dass**
die Polymermischung eine Mischung einer Poly-3,4-ethylendioxythiophen (PEDOT)-Verbindung mit einem auf Dimethacrylaten basierenden lichthärtenden Zahnzement umfasst.

2. Lichthärtende Polymermischung gemäß dem vorherigen Anspruch
**dadurch gekennzeichnet, dass**
die PEDOT-Verbindung mit dem auf Dimethacrylaten basierenden Zahnzement in einem Mischungsverhältnis zwischen 0.5:1 und 2:1, besonders bevorzugt in einem Mischungsverhältnis von 1:1 vorliegt.

3. Lichthärtende Polymermischung gemäß einem der vorherigen Ansprüche
**dadurch gekennzeichnet, dass**
die PEDOT-Verbindung ein Poly-3,4-ethylendioxythiophen Polystyrolsulfonat (PEDOT:PSS) ist.

4. Lichthärtende Polymermischung gemäß einem der vorherigen Ansprüche
**dadurch gekennzeichnet, dass**
die Polymermischung zusätzlich einen Polymerbinder, ein Tensid und/oder ein Lösungsmittel umfasst.

5. Lichthärtende Polymermischung gemäß einem der vorherigen Ansprüche
**dadurch gekennzeichnet, dass**
das auf Dimethacrylaten basierende Zahnzement Bisphenol-A-(di)-methacrylate (BIS-GMA), Urethandimethacrylat, Hexandioldimethacrylate (HDDMA) und/oder Triethylenglycoldimethacrylat umfasst.

6. Polymerelektrode zur Ableitung von Biosignalen von einer Haut eines Probanden umfassend eine lichthärtende Polymermischung gemäß einem der vorherigen Ansprüche.

7. Polymerelektrode zur Ableitung von Biosignalen von einer Haut herstellbar durch ein Verfahren umfassend
a. Bereitstellung einer lichthärtenden Polymermischung gemäß einem der vorherigen Ansprüche 1-5
b. Auftragen der lichthärtenden Polymermischung als dünnen Film auf die Haut
c. Härtung der lichthärtenden Polymermischung mittels einer Lichtbestrahlung,
wobei bevorzugt die Härtung mittels eine Lichtbestrahlung im Blaubereich, bevorzugt mittels einer Lichtbestrahlung in einem Wellenlängenbereich von 440 nm bis 480 nm erfolgt und/oder.
die Härtung mittels eine Lichtbestrahlung über einen Zeitraum von 5 - 60 Sekunden, bevorzugt von 10 - 20 Sekunden erfolgt.

8. Polymerelektrode gemäß einem der vorherigen Ansprüche 6 oder 7
**dadurch gekennzeichnet, dass**
die Polymerelektrode eine Impedanz von weniger gleich 10 kOhm, bevorzugt von weniger gleich 4 kOhm aufweist.

9. Polymerelektrode gemäß einem der vorherigen Ansprüche 6-8
**dadurch gekennzeichnet, dass**
innerhalb der Polymerelektrode ein oder mehrere leitfähige Fasern, bevorzugt ein oder mehrere leitfähige Kunststofffasern, eingebracht vorliegt.
, wobei
die Polymerelektrode bevorzugt herstellbar ist durch ein Verfahren umfassend.
a. Bereitstellung einer leifähigen Polymermischung gemäß einem der vorherigen Ansprüche 1-5
b. Bereitstellung ein oder mehrerer leitfähiger Fasern, bevorzugt ein oder mehrere leitfähige Kunststofffasern
c. Auftragen der leifähigen Polymermischung als dünnen Film auf die Haut
d. Einbringen der leitfähige Fasern, bevorzugt der leitfähigen Kunststofffasern, in die lichthärtende Polymermischung
e. Härtung der leifähigen Polymermischung mittels einer Lichtbestrahlung

10. Verwendung einer Polymerelektrode gemäß einem der Ansprüche 6-12 für die Ableitung elektrischer Biosignal, bevorzugt für die Elektroenzephalografie (EEG), die Elektrokardiographie (EKG) und/oder die Elektromyografie (EMG),
wobei bevorzugt die Ableitung der elektrischen Biosignale während einer Magnetresonanztomographie-Untersuchung erfolgt.

11. Vorrichtung zur Applikation einer Polymerelektrode gemäß einem der Ansprüche 6-9 auf einer Haut umfassend
- einen Spritzenkörper mit einem Spritzenauslauf und einem Spritzenbehälter eingerichtet zur Aufnahme einer leifähigen Polymermischung gemäß einem der vorherigen Ansprüche 1-5
- einen Kolbenaufsatz mit einem Spritzenkolben, welcher für die Aufnahme in den Spritzenbehälter und den Ausstoß der lichthärtenden Polymermischung durch den Spritzenauslauf konfiguriert ist
**dadurch gekennzeichnet, dass**
eine oder mehrere Lichtquellen um den Spritzenauslauf angeordnet vorliegen und eine Steuereinheit auf Basis eine Auslösesignales eine Aktivierung der Lichtquellen für eine Beleuchtungszeit steuert.

12. Vorrichtung gemäß dem vorherigen Anspruch
**dadurch gekennzeichnet, dass**
die Vorrichtung einen Sensor zur Detektion einer Bewegung des Spritzkolbens umfasst und das Auslösesignal zur Aktivierung der Blaulichtquellen einer detektierten Bewegung des Spritzkolbens entspricht, wobei bevorzugt
die Beleuchtungszeit zwischen 5 - 60 Sekunden, bevorzugt zwischen 10 - 20 Sekunden beträgt.

13. Vorrichtung gemäß einem der vorherigen Ansprüche
**dadurch gekennzeichnet, dass**
der Kolbenaufsatz eine Aufsatzhülse zur Führung des Spritzkolben, einen Druckknopf zur Steuerung des Spritzkolbens und eine Rückstellfeder für den Spritzkolben umfasst, wobei bevorzugt der Druckknopf zwei Zangenpaare umfasst, wobei ein Führungszangenpaar der Führung des Druckknopfes in der Aufsatzhülse und eine Steuerungszangenpaar zur Steuerung des Spritzkolbens dient.

14. Vorrichtung gemäß einem der vorherigen Ansprüche
**dadurch gekennzeichnet, dass**
der Spritzkolben eine Kolbenstange mit einer Zahnung aufweist, wobei die Zahnung in Bezug auf den Umfang der Kolbenstange bevorzugt in zwei gegenüberliegenden Bereichen vorliegt, welche von einem Bereich ohne Zahnung getrennt werden.

15. Kit umfassend
- eine Vorrichtung gemäß einem der vorherigen Ansprüche 11-14 und
- eine lichthärtende Polymermischung gemäß einem der Ansprüche 1-5
- optional Instruktionen zur Applikation der lichthärtenden Polymermischung mittels der Vorrichtung als Polymerelektrode auf der Haut.

## Claims

1. A light-curing polymer mixture for producing polymer electrodes
**characterized in that**
the polymer blend comprises a mixture of a poly-3,4-ethylenedioxythiophene (PEDOT) compound with a dimethacrylate-based light-curing dental cement.

2. The light-curing polymer mixture according to the preceding claim
**characterized in that**
the PEDOT compound with the dimethacrylate-based dental cement is present in a mixing ratio of between 0.5:1 and 2:1, particularly preferably in a mixing ratio of 1:1.

3. The light-curing polymer composition according to one or more of the preceding claims
**characterized in that**
the PEDOT compound is a poly-3,4-ethylenedioxythiophene polystyrene sulfonate (PEDOT:PSS).

4. The light-curing polymer composition according to one or more of the preceding claims
**characterized in that**
the polymer mixture additionally comprises a polymer binder, a surfactant and/or a solvent.

5. The light-curing polymer composition according to one or more of the preceding claims
**characterized in that**
the dimethacrylate-based dental cement comprises bisphenol A (di)methacrylates (BIS-GMA), urethane dimethacrylate, hexanediol dimethacrylates (HDDMA) and/or triethylene glycol dimethacrylate.

6. A polymer electrode for deriving biosignals from a skin of a subject comprising a light-curing polymer mixture according to one or more of the preceding claims.

7. A polymer electrode for deriving biosignals from a skin producible by a method comprising
a. providing a light-curing polymer mixture according to one of the preceding claims 1-5
b. applying the light-curing polymer mixture as a thin film to the skin
c. curing the light-curing polymer mixture by means of light irradiation,
wherein curing preferably takes place by means of light irradiation in the blue range, preferably by means of light irradiation in a wavelength range from 440 nm to 480 nm, and/or curing is effected by means of light irradiation over a period of 5-60 seconds, preferably 10-20 seconds.

8. The polymer electrode according to any one of the preceding claims 6 or 7
**characterized in that**
the polymer electrode has an impedance of less than or equal to 10 kOhm, preferably of less than or equal to 4 kOhm.

9. The polymer electrode according to any one of the preceding claims 6 or 8,
**characterized in that**
one or more conductive fibers, preferably one or more conductive plastic fibers, are introduced within the polymer electrode, wherein
the polymer electrode can preferably be produced by a process comprising
a. providing a conductive polymer mixture according to one of the preceding claims 1-5
b. providing one or more conductive fibers, preferably one or more conductive plastic fibers
c. applying the conductive polymer mixture as a thin film to the skin
d. introducing the conductive fibers, preferably the conductive plastic fibers, into the light-curing polymer mixture
e. curing the conductive polymer mixture by light irradiation.

10. The use of a polymer electrode according to one of Claims 6-12 for deriving electrical biosignal, preferably for electroencephalography (EEG), electrocardiography (ECG) and/or electromyography (EMG),
the electrical biosignals are preferably derived during a magnetic resonance imaging examination.

11. An apparatus for applying a polymer electrode according to any one of claims 6-9 to a skin, comprising
- a syringe body with a syringe outlet and a syringe barrel adapted to receive a conductive polymer mixture according to any one of the preceding claims 1-5
- a plunger head with a syringe plunger, which is configured for accommodation in the syringe barrel and ejection of the light-curing polymer mixture through the syringe outlet
**characterized in that**
one or more light sources are arranged around the syringe outlet and a control unit controls an activation of the light sources for an illumination time on the basis of a triggering signal.

12. The apparatus according to the preceding claim,
**characterized in that**
the apparatus comprises a sensor for detecting a movement of the syringe plunger and the triggering signal for activating the blue light sources corresponds to a detected movement of the injection plunger, wherein preferably
the illumination time is between 5-60 seconds, preferably between 10-20 seconds.

13. The apparatus according to any one of the preceding claims, **characterized in that**
the plunger attachment comprises an attachment sleeve for guiding the syringe plunger, a pushbutton for controlling the syringe plunger and a return spring for the syringe plunger, wherein the pushbutton preferably comprises two pairs of pincers, wherein a pair of guide pincers serves for guiding the pushbutton in the attachment sleeve and a pair of control pincers serves for controlling the injection piston.

14. The apparatus according to any one of the preceding claims, **characterized in that**
the syringe plunger has a piston rod with toothing, wherein the toothing is preferably present in two opposite regions with respect to the circumference of the piston rod, which regions are separated from a region without toothing.

15. A kit comprising
- an apparatus according to any one of the preceding claims 11-14 and
- a light-curing polymer blend according to any one of claims 1-5
- optionally instructions for the application of the light-curing polymer mixture by means of the apparatus as a polymer electrode on the skin.

## Revendications

1. Mélange polymère photodurcissable pour la fabrication d'électrodes polymères
**caractérisé en ce que**
le mélange polymère comprend un mélange d'un composé de poly-3,4-éthylènedioxythiophène (PEDOT) et d'un ciment dentaire photodurcissable à base de diméthacrylates.

2. Mélange polymère photodurcissable selon la revendication précédente
**caractérisé en ce que**
le composé PEDOT avec le ciment dentaire à base de diméthacrylates est présent dans un rapport de mélange compris entre 0,5:1 et 2:1, de préférence dans un rapport de mélange de 1:1.

3. Mélange polymère photodurcissable selon l'une des revendications précédentes
**caractérisé en ce que**
le composé PEDOT est un poly-3,4-éthylènedioxythiophène polystyrène sulfonate (PEDOT:PSS).

4. Mélange polymère photodurcissable selon l'une des revendications précédentes
**caractérisé en ce que**
le mélange polymère comprend également un liant polymère, un tensioactif et/ou un solvant.

5. Mélange polymère photodurcissable selon l'une des revendications précédentes
**caractérisé en ce que**
le ciment dentaire à base de diméthacrylates comporte des (di)méthacrylates de bisphénol A (BIS-GMA), du diméthacrylate d'uréthane, des diméthacrylates d'hexanediol (HDDMA), et/ou du diméthacrylate de triéthylèneglycol.

6. Électrode polymère pour la dérivation de biosignaux à partir de la peau d'un sujet, comprenant un mélange polymère photodurcissable selon l'une des revendications précédentes.

7. Électrode polymère pour la dérivation de biosignaux à partir d'une peau pouvant être fabriquée par un procédé comprenant
a. la préparation d'un mélange polymère photodurcissable selon l'une des revendications précédentes 1 à 5
b. l'application du mélange polymère photodurcissable sous la forme d'un film mince sur la peau
c. le durcissement du mélange polymère photodurcissable par irradiation lumineuse,
dans laquelle le durcissement s'effectue de préférence au moyen d'une irradiation lumineuse dans la zone bleue, de préférence au moyen d'une irradiation lumineuse dans une plage de longueurs d'onde de 440 nm à 480 nm et/ou.
le durcissement s'effectue au moyen d'une irradiation lumineuse pendant une période de 5 à 60 secondes, de préférence de 10 à 20 secondes.

8. Électrode polymère selon l'une des revendications précédentes 6 ou 7
**caractérisée en ce que**
l'électrode polymère présente une impédance inférieure ou égale à 10 kOhms, de préférence inférieure ou égale à 4 kOhms.

9. Électrode polymère selon l'une des revendications précédentes 6 à 8
**caractérisée en ce que**
une ou plusieurs fibres conductrices, de préférence une ou plusieurs fibres conductrices en matière plastique, sont incorporées dans l'électrode polymère,
dans laquelle
l'électrode polymère peut de préférence être fabriquée par un procédé comprenant.
a. la préparation d'un mélange polymère conducteur selon l'une des revendications précédentes 1 à 5
b. la fourniture d'une ou de plusieurs fibres conductrices, de préférence une ou plusieurs fibres conductrices en matière plastique
c. l'application du mélange polymère conducteur sous forme de film mince sur la peau
d. l'introduction des fibres conductrices, de préférence des fibres conductrices en matière plastique, dans le mélange polymère photodurcissable
e. le durcissement d'un mélange polymère conducteur par irradiation lumineuse

10. Utilisation d'une électrode polymère selon l'une des revendications 6 à 12 pour la dérivation de biosignaux électriques, de préférence pour l'électroencéphalographie (EEG), l'électrocardiographie (ECG) et/ou l'électromyographie (EMG),
dans laquelle la dérivation des biosignaux électriques s'effectue de préférence pendant un examen par imagerie par résonance magnétique.

11. Dispositif pour appliquer une électrode polymère selon l'une des revendications 6 à 9 sur la peau, comprenant
- un corps de seringue avec une sortie de seringue et un réservoir de seringue agencé pour recevoir un mélange polymère conducteur selon l'une des revendications précédentes 1 à 5
- un embout de piston avec un piston de seringue configuré pour être reçu dans le réservoir de la seringue et pour évacuer le mélange polymère photodurcissable à travers la sortie de la seringue
**caractérisé en ce que**
une ou plusieurs sources lumineuses sont disposées autour de la sortie de la seringue et une unité de commande commande l'activation des sources lumineuses pendant un temps d'éclairage à partir d'un signal de déclenchement.

12. Dispositif selon la revendication précédente
**caractérisé en ce que**
le dispositif comprend un capteur pour détecter un mouvement du piston de seringue et le signal de déclenchement pour activer les sources de lumière bleue correspond à un mouvement détecté du piston de seringue, dans lequel de préférence le temps d'éclairage est compris entre 5 et 60 secondes, de préférence entre 10 et 20 secondes.

13. Dispositif selon l'une des revendications précédentes,
**caractérisé en ce que**
l'embout de piston comprend un manchon pour guider le piston de seringue, un bouton-poussoir pour commander le piston de seringue et un ressort de rappel pour le piston de seringue, dans lequel le bouton-poussoir comprend de préférence deux paires de pinces, dans lequel une paire de pinces de guidage sert à guider le bouton-poussoir dans le manchon et une paire de pinces de commande sert à commander le piston de seringue.

14. Dispositif selon l'une des revendications précédentes
**caractérisé en ce que**
le piston de seringue présente une tige de piston avec une denture, dans lequel la denture est de préférence présente dans deux zones opposées par rapport à la périphérie de la tige de piston, lesquelles sont séparées d'une zone sans denture.

15. Kit comprenant
- un dispositif selon l'une des revendications précédentes 11 à 14 et
- un mélange polymère photodurcissable selon l'une des revendications 1 à 5
- des instructions facultatives pour l'application du mélange polymère photodurcissable sur la peau au moyen du dispositif en tant qu'électrode polymère.
